# EUROPÄISCHE PATENTSCHRIFT

(11) **EP 0 689 430 B1**
(45) Veröffentlichungstag und Bekanntmachung des Hinweises auf die Patenterteilung: **13.08.1997**
(21) Anmeldenummer: 94912435.8
(22) Anmeldetag: 17.03.1994
(51) Int. Cl.: A61K 9/50, A61K 49/00

(54) **SUPERPARAMAGNETISCHE TEILCHEN, VERFAHREN ZU IHRER HERSTELLUNG UND VERWENDUNG DERSELBEN**
SUPERPARAMAGNETIC PARTICLES, PROCESS FOR PRODUCING THE SAME AND THEIR USE
PARTICULES SUPRAPARAMAGNETIQUES, LEUR PROCEDE DE PRODUCTION ET LEUR UTILISATION

(30) Priorität: 17.03.1993 DE 4309333; 02.03.1994 DE 4407338
(43) Veröffentlichungstag der Anmeldung: 03.01.1996
(73) Patentinhaber: SILICA GEL GES.M.B.H, D-14050 Berlin (DE); Pilgrimm, Herbert Dr., 14169 Berlin (DE)
(72) Erfinder: PILGRIMM, Herbert, D-14169 Berlin (DE)
(74) Vertreter: Walter, Wolf-Jürgen
(86) Internationale Anmeldenummer: DE9400314
(87) Internationale Veröffentlichungsnummer: WO9421240

(56) Entgegenhaltungen:
- EP-A- 0 125 995
- EP-A- 0 284 549
- EP-A- 0 516 252
- WO-A-88/00060
- WO-A-90/01295
- WO-A-90/01899
- US-A- 5 069 216
- MAGMA, Bd.1, Nr.2, 1993 Seiten 83 - 88 A ROCH ET AL. 'IN VITRO RELAXOMETRIC CHARACTERIZATION OF SUPERPARAMAGNETIC CONTRAST AGENTS'

## Beschreibung

Die Erfindung betrifft superparamagnetische Teilchen, die auf ihrer Oberfläche Substanzen chemisch gebunden haben, die gegebenenfalls weitere Bindungsstellen zur Kopplung von gewebespezifischen Bindungssubstanzen, diagnostischen oder pharmakologisch wirksamen Substanzen besitzen sowie neue damit in Zusammenhang stehende Verbindungen und die Verwendung dieser Teilchen und Verbindungen in der Medizin zur Tumorschädigung, Immunsteigerung und Diagnostik.

Magnetische Teilchen sind in einer Vielzahl von Veröffentlichungen und Patenten beschrieben worden, vor allen Dingen für die magnetische Separationstechnik und für den Einsatz als Kontrastmittel in der NMR-Diagnostik.

In den 1960'er Jahren versuchte man ferromagnetischen Teilchen als Kontrastmittel für die Röntgendiagnostik und für ein magnetisch kontrolliertes drug targeting einzusetzen. So z.B. MEYERS,P.H. et al. J. Am. J. Roentgenol. Radium Ther. Nucl. Med., 90,1068,1963; Frei,F.H. et al. J. Appl. Phys.,39,999,1968; Nakamura et al. J. Appl. Phys.,42,1320,1971; Hier erwies sich die irreversible Aggregation der Magnetteilchen unter der Wirkung eines Magnetfeldes als Problem bei der in vivo Anwendung. Ähnliches gilt für die DE-A-3590398, US-A-4,675,173, US-A-4,615,879, WO 84/02643, GB-A-8408127 und WO 84/04330. Hier werden ferromagnetische Teilchen mit Weiß'schen Bezirken in der Größenordnung von einigen Hundert bis einigen Tausend Ångströmeinheiten vorgeschlagen, die mit einer Polymerbeschichtung versehen wurden, um Substanzen mit Bindeaffinität für Gewebe koppeln zu können.

Ferromagnetische Teilchen in der vorgeschlagenen Größe haben so große magnetische Momente, daß die Teilchen sich zu größeren Aggregaten zusammenlagern, auch wenn sie mit einer Polymerbeschichtung versehen werden. Schon während der Beschichtung liegen die Teilchen aggregiert vor. Solche ferromagnetischen Teilchen würden bei parenteraler Anwendung im Körper sedimentieren, die toxischen Nebenwirkungen groß sein.

Ähnliche Nachteile treffen auch für die in der DE-A-3443251 und DE-A-3443252 verwendeten Dispersionen von ferromagnetischen Teilchen zu, wo die magnetischen Wechselwirkungen zwischen den Teilchen zu Aggregation und Sedimentation führen. Die irreversible Sedimentation der Magnetteilchen erfolgt besonders bei der Einwirkung von Magnetfeldern sehr schnell. Treten Inhomogenitäten des Magnetfeldes auf, konzentrieren sich die Magnetteilchen immer an den Stellen hoher Feldstärken. Diese Nachteile treten besonders bei NMR-Diagnoseverfahren und beim magnetischen drug targeting auf, wobei es zur Ausbildung sehr großer irreversibler Teilchenaggregate kommen kann, die eine große Emboliegefahr bedeuten.

Noch größere ferromagnetische Teilchen sind in US-A-3,933,997, US-A-3,652,761, Nature 270,259,1977, J. Allergy Clin. Immunol. 61,23,1978 , US-A-4,177,253, Clin. Chem.,26,730,1980, Clin. Chem., 26,1281,1980, US-A-3,970,518, US-A-4,230,685, US-A-4,267,234, US-A-4,152,210, US-A-4,343,901 beschrieben. Diese Magnetteilchen mit Durchmesser zwischen 10 und 160 µm lassen sich schon mit schwachen Magnetfeldern abscheiden, haben aber den Nachteil, daß sie sehr schnell sedimentieren, eine kleine spezifische Oberfläche für die Bindung von pharmakologisch aktiven Substanzen besitzen, im Magnetfeld irreversibel agglomerieren und für drug targeting wegen der Emboliegefahr zu groß sind.

Die irreversible Agglomeration der Magnetteilchen im Magnetfeld läßt sich verhindern, in dem superparamagnetische Teilchen angewendet werden. Superparamagnetische Teilchen besitzen keine Remanenz, d.h. sie lassen sich in einem magnetischen Gradientenfeld reversibel bewegen und konzentrieren. Solche superparamagnetischen Teilchen sind z.B. Eisenoxide mit einem Teilchendurchmesser kleiner 0,02µm.

Damit diese superparamagnetischen Teilchen in wäßrigen Dispersionen nicht sedimentieren, werden Stabilisatorsubstanzen zugegeben, die sich adsorptiv auf den Teilchenoberflächen anlagern. Solche Teilchen sind in US-A-3,215,572, US-A-3,531,413, US-A-3,917,538, WO 85/02772, US-A-4,101,435 und US-A-4,452,773, SE-A-8307060-7 beschrieben.

Die adsorptionsstabilisierten Magnetteilchen sind unter physiologischen Bedingungen nicht stabil, da durch Ablösung der Stabilisatorsubstanzen die Magnetteilchen leicht aggregieren. Werden an adsorptionsstabilisierte Magnetteilchen Substanzen mit Bindeaffinität für bestimmte Gewebe oder pharmakologischer Wirkung gekoppelt, so besteht die Gefahr, daß die Stabilisatorsubstanzen und somit die Substanzen mit Bindeaffinität und pharmakologischer Wirkung von den Magnetteilchen abgelöst werden und die Magnetteilchen den Bindungsort nicht erreichen oder bei magnetischem drug targeting die pharmakologisch wirksame Substanz am Wirkungsort nicht angereichert wird.

Aus der WO 90/01889 sind Aggregate von bioabbaubaren superparamagnetischen Metalloxidkristallen bekannt, bei denen durch Zugabe von polyfunktionellen Substanzen die einzelnen superparamagnetischen Kristalle mit den polyfunktionellen Substanzen Aggregate bilden. Die Herstellung derartiger Aggregate entspricht der Verfahrensweise in der WO 88/00060, wonach die Eisenoxidteilchen aus einer Lösung, die zusätzlich eine polyfunktionelle Substanz, wie Polysaccharid, Protein usw. enthält, im basischen Medium zu z.B. Polysaccharid-überzogenen superparamagnetischen Eisenoxidteilchen ausgefällt, danach dialysiert und schließlich zentrifugiert werden. Dabei kommt es zu einer Aggregation der ausgefällten Teilchen.

Die WO 90/01295 betrifft MR-Kontrastmittel aus superparamagnetischen Metalloxiden in Assoziation mit makromolekularen Verbindungen oder Konjugaten dieser Moleküle mit anderen polymeren Substanzen, die auf ähnliche Weise wie die der WO 88/00060 hergestellt werden.

In der US-A-5069216 wird ein Verfahren zum Erhalten von in vivo MR-Bildern des Magen-Darm-Traktes beschrieben, bei dem die superparamagnetischen Aggregate der WO 90/01295 bzw. WO 88/00060 eingesetzt werden. Die EP-A-125 995 betrifft Magnetteilchen mit einem magnetischen Metalloxidkern mit einem Silanüberzug, an den Moleküle kovalent angekoppelt werden können.

Roch, A. et al haben in Magma 1 (1993) 83-88 superparamagnetische Kontrastmittel beschrieben, die aus Agglomeraten bestehen, deren Herstellung in üblicher Weise durch Zusatz von z.B. Dextran zu Eisensalzlösungen erfolgt.

Die EP-A-516 252 beschreibt nanokristalline Magnetpartikel mit einer Umhüllung aus chemisorbierten Glycosaminoglycanen, die aus Eisensalzen über eine biomimetische Synthese im Neutralbereich bei Umgebungstemperatur und ohne Ultraschallbehandlung abläuft.

In der EP-A-0284549 enthalten die Stabilisatorsubstanzen Phosphat- oder Phosphonatgruppen, über die sie mit der Oberfläche der superparamagnetischen Teilchen chemisch verbunden sind. Enthalten die Stabilisatorsubstanzen noch chemisch reaktive Gruppen, können pharmakologisch wirksame Substanzen gekoppelt werden. Diese chemisch stabilisierten superparamagnetischen Teilchen sedimentieren nicht in wäßrigen Dispersionen, sie haben nur einen Durchmesser von 0,003 bis 0,01µm. Bei parenteraler Anwendung erfolgt keine Ablösung der Stabilisatorsubstanzen, d.h. es erfolgt keine Aggregation und Sedimentation im Blut und damit eine gute Verteilung im Organismus.

Für ein magnetisches drug targeting sind diese Magnetteilchen zu klein, da sehr große Magnetfeldgradienten eingesetzt werden müßten, um eine Anreicherung der Magnetteilchen in bestimmte Körperregionen zu erreichen.

Größere superparamagnetische Teilchen lassen sich durch Einbetten von kleinen, 0,01µm großen superparamagnetischen Teilchen in poröse Polymerteilchen (SE-A-7706431, Polyglutaraldehyd-Polymere (US-A-4,267,234), Silan-Polymere (US-A-4,554,088), Albumin-Kondensations-Polymere (US-A-4,675,173) oder Celluloseester (Ito,R.,et al., Int.J. Pharm., 61,109,1990), herstellen. Die Teilchendurchmesser liegen im Bereich von 0,05 bis 100µm. Bis auf die Albumin-Kondensations-Polymere und die Celluloseester sind alle verwendeten Polymerisationschemikalien physiologisch bedenklich und die Auflösegeschwindigkeit der Magnetteilchen im Körper sehr klein. Alle genannten größeren superparamagnetischen Teilchen sedimentieren im Schwerefeld der Erde, sie müssen deshalb vor der Benutzung wieder dispergiert werden.

Die Eisenoxidgehalte der Polymerteilchen liegen im Bereich von 10 bis maximal 50 Gew.%, der Volumenanteil bei maximal 10 Vol.%.
Je kleiner die Magnetteilchen sind, desto höhere Magnetfeldgradienten benötigt man beim magnetischen drug targeting, um die Magnetteilchen in bestimmte Körperregionen zu konzentrieren. Je größer die Magnetteilchen sind, desto schneller werden sie vom retikuloendothelialem System gebunden, d.h. die Bioverfügbarkeit verkürzt sich und um so kleiner ist der Anteil an gebundener pharmakologische wirksamer Substanz. Optimale Magnetteilchen für ein magnetisches drug targeting sollten demzufolge möglichst klein sein, eine möglichst große Beladung mit pharmakologisch wirksamen Substanzen, einen möglichst hohen Anteil an Magnetmaterial, eine möglichst große magnetische Permeabilität des Magnetmaterials, eine möglichst große Auflösungsgeschwindigkeit im Körper, und eine ausreichende Bioverträglichkeit im Körper besitzen.

Der in Anspruch 1 angegeben Erfindung liegt die Aufgabe zugrunde, neue Verbindungen und superparamagnetische Teilchen herzustellen, um die eingangs genannten Nachteile zu vermeiden und neue Anwendungsgebiete insbesondere bei der Tumorbekämpfung und der Immunsteigerung zu erschließen.

Erfindungsgemäß wird das dadurch erreicht, daß sehr kleine superparamagnetische Eindomänenteilchen zur Aggregation gebracht werden und durch eine chemische Bindung von reaktiven Stabilisatorsubstanzen auf der Oberfläche der superparamagnetischen Teilchen vor einer weiteren Aggregation geschützt werden. Dabei können sowohl die neuen superparamagnetischen Aggregate als auch die reaktiven Stabilisatorsubstanzen im erfindungsgemäßen Sinne wirksame Substanzen sein.

Es erfolgt somit im Gegensatz zum Stand der Technik zuerst eine Aggregation der Eindomänenteilchen und anschließend die Bindung der Stabilatorsubstanzen an die Aggregatoberfläche, wodurch sich andere Eigenschaften der Aggregate ergeben.

Die erfindungsgemäßen superparamagnetische Teilchen, bestehend aus Eindomänenteilchen und Stabilisatorsubstanzen, sind daher dadurch gekennzeichnet, daß superparamagnetische Eindomänenteilchen aus Eisenoxid-, Eisenmischoxid- oder Eisen mit einer Teilchengröße im Bereich zwischen 3 und 20 Nanometer zu stabilen, abbaubaren, ein definiertes Verhalten im Magnetfeld zeigenden Aggregaten zusammengelagert sind, mit einer Teilchengröße der Aggregate im Bereich zwischen 10 und 1000 Nanometer, wobei die Aggregate auf ihrer Oberfläche eine monomolekulare Schicht aufweisen, bestehend aus Stabilisatorsubstanzen der Gruppe der phosphat-, diphosphat-, carboxylat-, polyphosphat-, thiophosphat-, phosphonat-, thiophosphonat-, sulfat-, sulfonat-, mercapto-, silantriol-, trialkoxysilangruppenhaltigen Polyalkylenglykole, der Kohlehydrate oder der phos-phatgruppenhaltigen Nucleotide, deren Oligomere oder deren Polymere, die weitere Bindungsstellen haben können.

Es ist vorteilhaft die superparamagnetischen Eindomänenteilchen so klein als möglich herzustellen, um die biologische Abbaubarkeit hoch und die Toxizität möglichst gering zu halten. Die superparamagnetischen Eindomänenteilchen liegen dabei im Durchmesserbereich von 0.001 bis 0,02µm, vorzugsweise im Bereich von 0,003 bis 0,01µm.

Als physiologisch verträgliche Materialien für den in vivo Einsatz kommen z.B. γ-Fe₂O₃, Fe₃O₄ und Fe zur Anwendung. Für in vitro Anwendungen sind auch toxischere Magnetmaterialien einsetzbar, wie Eisenmischoxide der allgemeinen Formel MO•Fe₂O₃3, worin M die zweiwertigen Metallionen Fe, Mg, Be, Mn, Zn, Co, Ba, Sr, Cu oder Gemische davon bedeuten oder wie Eisenmischoxide der allgemeinen Formel mFe₂O₃ • nMe₂O₃, worin Me die dreiwertigen Metallionen Al, Cr, seltene Erdmetalle oder Gemische davon bedeuten.

Erfindungsgemäß werden die superparamagnetischen Eindomänenteilchen durch eine thermische Behandlung in wäßriger Dispersion unter Änderung des pH-Wertes, der Temperatur und gegebenenfalls des Druckes, zur Aggregation gebracht. Überraschend wurde gefunden, daß sich die superparamagnetischen Eindomänenteilchen bei pH-Änderung der Fällungsdispersion von 8,0 bis 10,0 auf pH 3,0 bis 7,0 und bei einer Erwärmung auf Temperaturen zwischen 50 und 120°C zu größeren superparamagnetische Teilchen zusammenlagern. Überraschend findet kein Kristallwachstum statt, was zu ferromagnetischen Teilchen führen würde, sondern nur eine Aggregation der Eindomänenteilchen zu größeren Teilchenverbänden, so daß der superparamagnetische Charakter der Aggregate erhalten bleibt. Dabei ist die Stabilität der Aggregate so groß, daß durch die anschließende Zugabe der Stabilisatorsubstanzen sich nur ein geringer Anteil an stabilisierten superparamagnetischen Eindomänenteilchen bildet. Je nach pH-Wert, Temperatur, Temperaturgradient, Aggregationszeit, Elektrolytart und Elektrolytkonzentration der wäßrigen Dispersion lassen sich unterschiedliche Teilchendurchmesser in engen Durchmesserbereichen herstellen. Je niedriger der pH-Wert der Dispersion eingestellt wird, desto größer werden die Aggregate. In die gleiche Richtung wirken eine Temperaturerhöhung und eine Verlängerung der Aggregationszeit. Bei zu langen Aggregationszeiten bilden sich vermehrt größere ferromagnetische Teilchen, die für die erfindungsgemäße Anwendung nicht geeignet sind. Auch die Elektrolytart und die Elektrolytkonzentration wirken über die Dicke der elektrochemischen Doppelschicht der superparamagnetischen Eindomänenteilchen auf die Teilchenaggregation ein.

Die herstellbaren Teilchendurchmesser liegen im Bereich von 0,01 bis 10 µm, vorzugsweise jedoch im Bereich von 0,02 bis 1µm, insbesondere im Bereich von 0,02 bis 0,5 µm. Die kleinen Teilchen sind wegen ihrer großen Oberfläche und der damit verbundenen größeren Wirkstoffbindung bevorzugt.

Erfindungsgemäß erfolgt die Stabilisierung der Magnetteilchen durch eine chemische Bindung von phosphathaltigen Resten, ausgewählt unter Monophosphat, Diphosphat, Polyphosphat, Phosphonat, Thiophosphat, Thiophosphonat, oder ein carboxylat-, sulfat-, sulfonat-, mercapto-, silantriol-oder trialkoxysilanhaltiger Rest zls Stabilisatorsubstanz auf der Oberfläche der superparamagnetischen Teilchen. Die Stabilisatorsubstanz muß so beschaffen sein, daß sie mit Wasser mischbar ist und den Magnetteilchenabstand so groß hält, daß die kinetische Energie der Magnetteilchen größer als die magnetische Wechselwirkungsenergie ist. Die Stahilisatorsubstanzen können ausgewählt werden unter den folgenden Substanzen:
(i) den Verbindungen der allgemeinen Formel

   X - R - A - B

   worin X eine funktionelle Gruppe darstellt, ausgewählt aus der Alkoxy-, Alkylamino- und Alkylthiogruppe, bei denen die Zahl der Kohlenstoffatome im Alkylteil dieser Gruppen im Bereich von 1 und 4 liegt, oder eine funktionelle Gruppe darstellt, ausgewählt unter der Hydroxyl-, Amin-, Aldehyd-, Dimethylacetal-, Diethylacetal Epoxy-, Thiol-, Carboxy-, 4,6-Dichlortriazin-, Hydroxamsäure-, Isocyanat-, Acylazid-, Anhydrid-, Diazoniumsalz-, Iminocarbonat- und Toluolsulfonatgruppe;
   R fehlt oder
   R ist ein Polyalkylenglykol, ein mit Wasser mischbarer Polypropylenglykolrest oder ein mit Wasser mischbarer Block-Copolymerisatrest aus Polyethylenglykol (PEG) und Polypropylenglykol (PPG), ausgewählt unter den Block-Copolymerisaten

   (PEG)ₐ-(PEG)_{b} , (PEG)ₐ-(PPG)_{b}-(PEG)ₐ, (PPG)_{b}-(PEG)ₐ-(PPG)_{b}

   wobei a eine positive ganze Zahl im Bereich von 1 bis 100 und b eine positive ganze Zahl im Bereich von 1 bis 20 darstellt;
   n ist eine positive ganze Zahl, ausgewählt für PEG im Bereich von 4 bis 300, für PPG im Bereich von 3 bis 12 und für PEG-PPG-Block-copolymerisat im Bereich von 3 bis 140; oder
   R ist ein Kohlehydratrest, ausgewählt aus den Monosacchariden Glucose, Fructose, Ribose, Desoxyribose, Inosit, aus den Oligosacchariden Saccharose, Raffinose, Gentianose, Malecitose, Stachyose, Verbascose, aus den Polysacchariden Stärke, Lichenine, Glykogen, Dextrine, Dextrane, Inuline, Fruktosane, Lävane, Mannane, Galaktane, Xylane, Arabane, Pektine, Makropolysaccharide, Glycoproteide, aus Polyuridenylsäure, Polyglucuronsäure, Polygalacturonsäure, Polymannuronsäure und/oder Alginsäure bestehen;
   A fehlt oder
   A ist eine Alkyl-, Alkoxy-, Acyl-, Acylamin-, Alkylamingruppe, bei denen die Zahl der Kohlenstoffatome der Alkoxy-, Acyl-, Acylamin-, Alkylgruppe im Bereich von 1 bis 4 liegt;
   B ist ein phosphorhaltiger Rest, ausgewählt unter Monophosphat, Diphosphat, Polyphosphat, Phosphonat, Thiophosphat, Thiophosphonat, oder ein carboxylat-, sulfat-, sulfonat-, mercapto-, silantriol-oder trialkoxysilanhaltiger Rest;
(ii) den phosphatgruppenhaltigen Nucleotiden Mono-, Di-, Tri-phosphorsäureester oder Mono-, Di-, Tri-phosphorsäureesterchloriden von Adenosin, Guanosin, Cytidin, Uridin, Thymidin, Desoxyadenosin, Desoxyguanosin, Desoxycytidin, Desoxythymidin, Inosin, Pyrimidin, Cytosin, Uracil, Thymin, Purin, Adenin, Guanin, Methylcytosin, 5-Hydroxymethyl-cytosin, 2-Methyladenin, 1-Methylguanin, Thiamin, Flavin, Riboflavin sowie Pyridoxalphosphat, Pyridoxaminphosphat, Ribonucleinsäure, Ribonucleinsäuresequenzen, Desoxyribonucleinsäuren, Desoxyribonucleinsäuresequenzen;
(iii) den silicatgruppenhaltigen Verbindungen der Orthokieselsäure und deren Kondensationsprodukte; und /oder
(iv) X - R - A - B ist Mercaptopurin, -cytosin, -guanin, -uracil, -thymin, -hypoxanthin, sowie deren Mercapto-nucleoside und deren Mercapto-desoxynucleoside;
(v) X - R - A - B ist ein Polyaminokohlehydrat.
   Beispielhafte Stabilisatorsubstanzen sind
(vi) Mono- und Di-[ω-Ethylamino-polyethylenglykol]-diphosphat [Molekulargewicht (MG) des PEG ca. 1500], Mono- und Di-[ω-Ethoxy-polyethylenglykol]-thiophosphat (MG des PEG ca.1000) oder Mono- und Di-[ω-Methoxy-polyethylenglykol-polypropylenglykol]-phosphat, hergestellt aus Polyglykol M41/40 (Handelsbezeichnung der Fa. Hoechst, Deutschland),
   Mono- und Di-[ω-Hydroxy-polyethylenglykol]-phosphat (MG PEG ca.1500), Mono- und Di-[ω-Oxoethoxy-polyethylenglykol]-phosphonat oder deren Acetale (MG PEG ca.2000), Mono- und Di-[ω-Oxoethylamino-polyethylenglykol]-phosphat oder deren Acetale (MG PEG ca. 750), Mono- und Di-[ω-Aminoalkoxy-polyethylenglykol]-thiophosphat (MG PEG ca. 1000), Mono- und Di-[ω-Hydroxy-polyethylenglykolpolypropylenglykol]-phosphat, hergestellt aus Synperonic F68 (Handelsbezeichnung von ICI, Großbritannien), Mono- und Di-[ω-Methoxy-polyethylenglykol]-diphosphat (MG PEG ca.1000), das Dimethylacetal von Mono-[ω-Oxoethoxy-polyethylenglykol]-diphosphat (MG PEG ca. 2000), Mono-[ω-Ethoxy-polyethylenglykol]-polyphosphat (MG PEG ca.2000) oder Mono-[ω-Methoxypolyethylen-glykolpolypropylenglykol-diphosphat, hergestellt aus Polyglykol M 41/40 (Hoechst, DE).

Diese reaktiven, polyalkylenglykolhaltigen Stabilisatorsubstanzen können mit ihren Hydroxyl-, Carbonyl- oder Aminogruppen auch zur Einführung anderer reaktiver funktionaler Gruppen, wie z.B. der Thiol-, Epoxy-, Carboxy-, 4,4,6-Dichlortriazin Hydroxamsäure-, Isocyanat-, Acylazid-, Anhydrid-, Diazoniumsalz-, Iminocarbonat-, Toluolsulfonatgruppen, verwendet werden, um andere Bindungsorte an den gewebespezifischen und pharmakologisch wirksamen Substanzen zu realisieren;
(ii) aus den phosphatgruppenhaltigen Nucleotiden Mono-, Di-, Tri-phosphorsäureestern oder Mono-, Di-, Tri-phosphorsäureesterchloriden von Adenosin, Guanosin, Cytidin, Uridin, Thymidin, Desoxyadenosin, Desoxyguanosin, Desoxycytidin, Desoxythymidin, Inosin, Pyrimidin, Cytosin, Uracil, Thymin, Purin, Adenin, Guanin, Methylcytosin, 5-Hydroxymethyl-cytosin, 2-Methyladenin, 1-Methylguanin, Thiamin, Flavin, Riboflavin sowie Pyridoxalphosphat, Pyridoxaminphosphat, Ribonucleinsäure, Ribonucleinsäuresequenzen, Desoxyribonucleinsäuren, Desoxyribonucleinsäuresequenzen;
(iii) aus den phosphat-, diphosphat-, polyphosphat- und thiophosphat-, carboxylat-, sulfat-, sulfonat-, mercapto-, silantriol- oder trialkoxysilangruppenhaltigen Kohlehydraten, wobei die Kohlehydratreste aus den Monosacchariden Glucose, Fructose, Ribose, Desoxyribose, Inosit, aus den Oligosacchariden Saccharose, Raffinose, Gentianose, Malecitose, Stachyose, Verbascose, aus den Polysacchariden Stärke, Lichenine, Glykogen, Dextrine, Dextrane, Inuline, Fruktosane, Lävane, Mannane, Galaktane, Xylane, Arabane, Pektine, Makropolysaccharide, Glycoproteide, aus Polyuridenylsäure, Polyglucuronsäure, Polygalacturonsäure, Polymannuronsäure und/oder Alginsäure bestehen; oder aus mehreren dieser Reste.

Erfindungsgemäß können an die Stabilisatorsubstanzen, die an die superparamagnetischen Teilchenoberfläche gebunden sind, gewebespezifische Bindungssubstanzen oder pharmakologisch wirksame Substanzen gekoppelt werden, wenn die Stabilisatorsubstanzen wenigstens zwei chemisch reaktive funktionelle Gruppen tragen, wobei die Phosphat-, Diphosphat-, Polyphosphat-, Thiophosphat-, Phosphonat-, Thiophosphonat-, Carboxylat-, Sulfat-, Sulfonat-, Mercapto-, Silantriol- oder Trialkoxysilangruppe für die chemische Bindung zu den superparamagnetischen Teilchen dienen und die restlichen reaktiven funktionellen Gruppen, die z.B. aus Hydroxyl-, Amin-, Aldehyd-, Epoxy-, Thiol-, Carboxy-, 4,6-Dichlortriazin-, Hydroxamsäure-, Isocyanat-, Acylazid-, Anhydrid-, Diazoniumsalz-, Iminocarbonat-,Toluolsulfonatgruppen bestehen, für die Bindung von gewebespezifischen Bindungssubstanzen und pharmakologisch wirksamen Substanzen dienen.

Solche Stabilisatorsubstanzen sind z.B. Mono-, Oligo- oder Polysaccharidphosphate, -carboxylate, -sulfate, -sulfonate, - thiole, -silantriole oder -trialkoxysilane, die vor oder nach der chemischen Bindung auf der superparamagnetischen Teilchenoberfläche mit den entsprechenden funktionellen Gruppen versehen werden. Unter die genannten Stabilisatorsubstanzen fallen auch die entsprechenden Polysäuren. Die Einführung der funktionellen Gruppen in die Stabilisatormoleküle ist bekannter Stand der Technik.

Außer diesen reaktiven Stabilisatorsubstanzen auf Kohlehydratbasis können reaktive phosphatgruppenhaltige Biomoleküle, wie z.B. Pyridoxalphosphat, Pyridoxaminphosphat oder Co-carboxylase;
mercaptogruppenhaltige Substanzen wie Mercaptopurin, -cytosin, - guanin, -uracil, -thymin, -hypoxanthin, sowie deren Mercapto-nucleoside und deren Mercapto-desoxynucleoside;
silantriol- oder trialkoxysilangruppenhaltige Substanzen wie ω-Ethylamino-polyethylenglykol-trimethoxysilan (MG des PEG ca. 1000), ω-Methoxy-polyethylenglykol-trimethoxysilan (MG des PEG ca.750) oder ω-Methoxy-polyethylenglykol-polypropylenglykolthioethyl-triethoxysilan, hergestellt aus Polyglykol M41/40 (Hoechst), ω-Hydroxy-polyethylenglykol-silantriol,3-Chlorpropyltri-methoxysilan, 2-Mercaptopropyl-triethoxysilan, 3-Aminopropyltriethoxysilan, 3-(2-Aminoethylamino)propyl-trimethoxysilan, Vinyl-triethoxysilan, Vinyl-tri(methoxyethoxy)silan, Methacryloxypropyl-trimethoxysilan, oder Poly-trialkoxysilyl-Stärke, Poly-trialkoxysilyl-Dextran, Poly-trialkoxysilyl-Dextrin;
sulfatgruppenhaltige Substanzen, wie Dextransulfat, Dextrinsulfat, Inulinsulfat;
carboxylatgruppenhaltige Substanzen, wie Polycarboxydextran, Polycarboxydextrin, Polycarboxyamylopektin; polyaminogruppenhaltige Substanzen, wie Polyaminodextran; silicatgruppenhaltigen Verbindungen der Orthokieselsäure und deren Kondensationsprodukte, wobei hier zur Stabilisierung der superparamagnetischen Teilchen Natrium- oder Kaliumsilikatlösung oder alkalische Lösungen von Alkalisilikaten mit kondensationsfähigen Hydroxoaluminaten;
sowie deren Polyverbindungen verwendet werden.

Erfindungsgemäß können an die Stabilisatormoleküle, die mit ihren Monophosphat-, Diphosphat-, Polyphosphat-, Phosphonat-, Thiophosphat-, Thiophosphonat-, Carboxylat-, Sulfat-, Sulfonat-, Mercapto-, Silantriol- oder Trialkoxysilangruppen mit der superparamagnetischen Teilchenoberfläche chemisch gebunden sind, gewebespezifischen Bindungssubstanzen, wie z.B. Antigene, Antikörper, Haptene, Protein A, Protein G, Endotoxin-bindende Proteine, Lectine, Selectine, gekoppelt werden.

Auch pharmakologisch wirksame Substanzen, wie z.B. Antitumorproteine, Enzyme, Antitumorenzyme, Antibiotika, Pflanzenalkaloide, Alkylierungsreagenzien, Antimetaboliten, Hormone und Hormonantagonisten, Interleukine, Interferone, Wachstumsfaktoren, Tumornekrosefaktoren, Endotoxine, Lymphotoxine, Urokinase, Streptokinase, Plasminogen-Streptokinase-Aktivator-Komplex, Gewebe-Plasminogen-Aktivatoren, Desmodus-Plasminogen-Aktivatoren, Makrophagen-Aktivierungskörper, Antisera, Proteaseninhibitoren, radioakiven Phosphor ³²P enthaltene Stabilisatorsubstanzen, Tenside oder pharmakologisch wirksame Zellen, wie z.B. Organellen, Viren, Mikroben, Algen, Pilze, insbesondere Erythrozyten, Thrombozyten, Granulozyten, Monozyten, Lymphozyten, Langerhans'sche Inseln oder pharmakologisch wirksame Komplexbildner aus der Gruppe der Polycarbonsäuren, Aminocarboxylsäuren, Porphyrinen, Katecholamine oder zellfusionsvermittelnde Substanzen
können an die reaktiven Stabilisatorsubstanzen einzeln oder nebeneinander gekoppelt werden.

Als zellfusionsvermittelnde Substanzen bewirken beispielsweise Polyethylenglykole in Konzentrationen über 25 Gew.-%, wie sie in noch höheren Konzentrationen in den Stabilisatorsubstanzschichten der superparamagnetischen Teilchen auftreten, Zellfusionen, was bei einer Anreicherung der polyethylenglykolhaltigen superparamagnetischen Teilchen in Tumoren zu einer weiteren Schädigung des Tumorgewebes führen kann.
Erfindungsgemäß können außer den Stabilisatorsubstanzen auch noch phosphat- oder phosphonatgruppenhaltige Arzneimittel chemisch auf der Oberfläche der superparamagnetischen Teilchen gebunden werden, wie z.B. Estramustin oder Diethylstilbestrol-diphosphat.

Die Toxizität solcher pharmakologisch wirksamen Substanzen kann dabei relativ hoch sein, da die Magnetteilchen sich aufgrund ihrer gewebespezifischen Wechselwirkung bevorzugt an den entsprechenden Bindungsorten anreichert oder durch magnetisches drug targeting zum Wirkungsort transportiert und angereichert werden. Die Dosis der pharmakologisch wirksamen Substanzen kann gering gehalten werden, da sich die Substanz am Wirkungsort konzentriert und der übrige Körper nur gering belastet wird.

Die Kopplung pharmakologisch wirksamer Substanzen an die superparamagnetischen Teilchen hat weiterhin den Vorteil, daß über die Relaxationszeitverkürzung der Therapiefortschritt mit der Kernspin-Diagnostik beobachtet werden kann.

Die Herstellung der superparamagnetischen Teilchen erfolgt durch eine gezielte Agglomeration von superparamagnetischen Eindomänenteilchen. Dabei werden die superparamagnetischen Eindomänenteilchen in Wasser verrührt und bei einem pH-Wert von 3 bis 7 durch Erhitzen auf 50 bis 120°C, bei Temperaturen über 100°C im Autoklaven, zur Aggregation gebracht. Nach dem Abkühlen der Dispersion werden die Teilchen so lange gewaschen, bis die elektrische Leitfähigkeit des Filtrates < 10 µS/cm beträgt. Die so hergestellten superparamagnetischen Teilchen bilden sofort einen schnell sedimentierenden Niederschlag, der sich auch durch starkes Rühren oder durch Ultraschallbehandlung nicht in eine stabile Dispersion überführen läßt. Erst die chemische Bindung von phosphat-, diphosphat-, polyphosphat-, thiophosphat-, phosphonat, carboxylat-, sulfat-, sulfonat-, thiol-, silantriol- oder trialkoxysilangruppenhaltigen Stabilisatorsubstanzen auf der Oberfläche der superparamagnetischen Teilchen sorgt für eine schnelle Dispergierung, bei einigen Stabilisatorsubstanzen sogar schon bei leichtem Rühren mit dem Glasstab.

Je nach Anwendungsgebiet können die magnetischen Dispersionen dialysiert werden, um den überschüssigen Anteil an Stabilisatorsubstanz zu entfernen. Vor allen Dingen bei chemisch reaktiven superparamagnetischen Teilchen, die zur Kopplung von gewebespezifischen Bindungssubstanzen und pharmakologisch wirksamen Substanzen verwendet werden sollen ist eine Dialyse notwendig.

Die stabilisierten superparamagnetischen Teilchendispersionen enthalten die noch nicht oder nur schwach aggregierten superparamagnetischen Eindomänenteilchen. Diese bilden eine stabile magnetische Flüssigkeit, die sich leicht von den größeren superparamagnetischen Teilchen durch eine Sedimentation in einem Magnetfeld entspechender Stärke und Inhomogenität abtrennen lassen.

In einer einfachen Ausführung der magnetischen Separation stellt man ein Becherglas mit der magnetischen Dispersion auf einen Permanentmagneten mit einer magnetischen Flußdichte von 0,1 mT und gießt nach einer Sedimentationszeit von ca. 30 min die überstehende magnetische Flüssigkeit ab. Zurück bleiben die superparamagnetischen Teilchen, die, je nach Teilchengröße, sich wieder spontan in der Dispersion verteilen oder als Bodensatz im Becherglas zurück bleiben. Bis zu Teilchengrößen von ungefähr 500 nm verteilen sich die superparamagnetischen Teilchen wieder spontan oder unter leichtem Rühren im wäßrigen Dispersionsmittel. Größere superparamagnetische Teilchen als ca. 500 nm können leicht durch stärkeres Rühren oder Ultraschallbehandlung dispergiert werden.

Die Sedimentationsstabilität der erfindungsgemäßen superparamagnetischen Teilchen ist wesentlich höher als bei den bisher bekannten Magnetteilchen mit vergleichbaren magnetischen Eigenschaften, was wahrscheinlich auf die starke Strukturierung der die superparamagnetischen Teilchen umgebenden Wassermoleküle und den damit vergrößerten Stokes'schen Teilchendurchmesser zurückzuführen ist.

Die magnetischen Eigenschaften der superparamagnetischen Teilchen sind aufgrund des geringen Anteils an Stabilisatorsubstanz stärker als bei den bisher bekannten. Da der Anteil an superparamagnetischen Eindomänenteilchen wesentlich höher als bei den bisher bekannten Magnetteilchen ist, ist auch die Abscheidungsgeschwindigkeit der superparamagnetischen Teilchen in einem inhomogenen Magnetfeld größer. In einer 10 Gew.-%igen wäßrigen Dispersion von superparamagnetischen Teilchen mit einem Durchmesser von ca 100 nm und einem Magnetitanteil von 95 % beträgt die Abscheidungszeit der Magnetteilchen auf einen Permanentmagneten mit einer magnetischen Flußdichte von 0,1 mT weniger als 1 min.

Die erfindungsgemäßen superparamagnetischen Teilchen haben Eisenoxidgehalte von 90 bis 98 Gew.-%. Gegenüber dem Stand der Technik, daß Magnetteilchen bis zu 50 Gew.-% Eisenoxid enthalten können, bedeutet das eine wesentliche Verbesserung der magnetischen Eigenschaften. Damit können die neuen superparamagnetischen Teilchen, bei gleichen magnetischer Wechselwirkung, entsprechend kleiner als die bisher bekannten Magnetteilchen sein. Die spezifische Oberfläche vergrößert sich, es können mehr pharmakologisch wirksame Substanzen oder gewebespezifische Bindungssubstanzen auf der Oberfläche gekoppelt werden. Mit Verkleinerung der Teilchengröße wird auch die biologische Verträglichkeit besser, die Abbaugeschwindigkeit im Körper erhöht. Auch die freie verfügbare Zeit der Magnetteilchen beim magnetischen drug targeting, d.h. die Zeit bis die Teilchen vom retikuloendothelialen System gebunden sind, erhöht sich mit Verringerung der Teilchengröße.

Die Bioverfügbarkeit der superparamagnetischen Teilchen im Körper beträgt nur wenige Minuten, d.h. das retikuloendotheliale System bindet die superparamagnetischen Teilchen sehr schnell. Makrophagen und neutrophilen Granulozyten erhalten magnetisierbare Eigenschaften, wenn sie sich an die superparamagnetischen Teilchen anheften. Voraussetzung für dieses Anheften ist, daß die Stabilisatorsubstanzen der superparamagnetischen Teilchen an die Rezeptoren der Makrophagen und den neutrophilen Granulozyten gebunden werden. Erfolgt solch eine Bindung, können die magnetisierten Makrophagen und neutrophilen Granulozyten mit Hilfe von Magnetfeldern bewegt werden. Die Makrophagen und die neutrophilen Granulozyten können die endotheliale Barriere in den Hochendothel-Venolen durchdringen und in das Gewebe eindringen. Dieser Prozeß kann bei magnetisierten Makrophagen und neutrophilen Granulozyten durch die Einwirkung eines Magnetfeldes unterstützt werden. Bei einem magnetischen drug targeting von Tumoren mit den erfindungsgemäßen superparamagnetischen Teilchen kann somit eine beschleunigte Anreicherung der magnetisierten Makrophagen und neutrophilen Granulozyten im Tumorgewebe erfolgen und eine immunologische Schädigung des Tumorgewebes einsetzen.

An der Phasengrenze zwischen den superparamagnetischen Teilchen und diamagnetischen Zellen treten mechanische Kräfte auf, die proportional der Permeabilitätsdifferenz der sich berührenden Materialien und dem Quadrat der wirkenden Magnetfeldstärke sind.

Da die erfindungsgemäßen Teilchen einen sehr hohen Anteil an superparamagnetischen Teilchen besitzen und die Stabilisatorsubstanz nur eine monomolekulare Schicht von wenigen Nanometern Dicke besitzt, treten an den Phasengrenzen zu diamagnetischen Zellen Kräfte auf, die zur Schädigung der Zellen führen können. Diese Kräfte können z.B. bei der Anreicherung der superparamagnetischen Teilchen in Tumoren, zu einer Tumorschädigung führen. Dieser magnetomechanische Effekt könnte auch dazu führen, daß bei einer starken Wechselwirkung zwischen der Stabilisatorsubstanz der superparamagnetischen Teilchen und dem Tumorgewebe, Oberflächenmoleküle des Tumorgewebes von den superparamagnetischen Teilchen gebunden und herausgezogen werden. Wenn diese superparamagnetischen Teilchen von den Makrophagen und den neutrophilen Granulozyten phagozytiert werden, können die Oberflächenmoleküle des Tumors als Antigen erkannt werden, auch wenn sie sonst nicht vom Immunsystem als Antigen identifiziert worden wären. Es kann eine mehr oder weniger starke Immunantwort des Körpers auf die Oberflächenmoleküle des Tumors und damit eine Schädigung des Tumors auftreten.

Eine Stimulierung des Immunsystems durch magnetomechanische Effekte kann auch dadurch erreicht werden, daß Viren, Bakterien oder Pilze in vitro mit reaktiven superparamagnetischen Teilchen gemischt werden. So können superparamagnetische Teilchen, die z.B. mit Mono-[ω-Oxoethoxy-polyethylen-glykol]-phosphat stabilisiert wurden, mit den Aminogruppen der Oberflächenproteine der Viren oder Bakterien kovalente chemische Bindungen eingehen. Durch Einwirkung starker inhomogener Magnetfelder oder mit Hilfe bekannter Zellaufschlußmethoden lassen sich so Oberflächenmoleküle der Viren-, Bakterien- oder Pilzoberflächen mit den superparamagnetischen Teilchen herstellen. Diese superparamagnetischen Proteinbruchstücke werden beim Spritzen in den Körper vom retikulendothelialen System aufgenommen, die Proteinbruchstücke als Antigen erkannt und der Körper reagiert mit einer entsprechenden Immunantwort, unter anderem mit einer Produktion von entsprechenden Antikörpern. So läßt sich auch eine Immunantwort des Körpers von Proteinen stimulieren, die für sich alleine vom Körper nicht als Antigen erkannt werden. Das hat besondere Bedeutung bei der Erzeugung einer Immunabwehr des Körpers gegen Tumorzellen, Viren, Bakterien, Hefen oder Pilze, für die es noch keine geeignete Chemotherapie gibt oder bei denen sich eine Resistenz gegen bekannte angewendete Arzneimittel herausgebildet hat.

Durch die Kopplung von reaktiven superparamagnetischen Teilchen an die Oberflächenmoleküle von z.B. Tumorzellen, Viren, Bakterien, Hefen, Pilzen und Heraustrennen dieser Oberflächenmoleküle durch starke inhomogene Magnetfelder oder bekannte Zellaufschlußverfahren lassen sich immunstimulierende superparamagnetische Molekülkomplexe gewinnen, die bei in vivo Anwendung zur Immunstimulierung und Schädigung dieser biologischen aktiven Zellen führen können.

Die diphosphat- oder polyphosphathaltigen Stabilisatorsubstanzen auf der Basis von Polyalkylenglykolen sind neue Verbindungen und besitzen selbst eine tumorschädigende Wirkung.

So werden bei der in vivo Anwendung dieser Stabilisatorsubstanzen Tumore von Mäusen geschädigt. Durch eine Kopplung dieser Stabilisatorsubstanzen an die superparamagnetischen Teilchen läßt sich die tumorschädigende Wirkung verstärken, indem durch ein magnetisches drug targeting die Konzentration der Stabilisatorsubstanz im Tumor wesentlich erhöht. Neben einer Verstärkung der tumorschädigenden Wirkung durch das magnetische drug targeting ist auch eine erhebliche Verkürzung der Wirkungszeit, bis zur beginnenden sichtbaren Tumorschädigung zu beobachten.

Die Stabilisatorsubstanzen sind nach dem Stand der Technik leicht herzustellen.

Die Herstellung der Polyethylenglykole erfolgt durch Oxethylierung einer reaktiven organischen Ausgangsverbindung, wie z.B. 2-Methoxyethanol, Aminoacetaldehyd-dimethylacetal, 2-Methoxy-ethylamin, 2-Aminoethanol mit Ethylenoxid.
Siehe **Houben-Weyl**, Bd. XIV/2, S.425 ff. (1963)

Die Einführung der Phosphat-, Diphospat-, Polyposphat- oder Thiophosphatgruppe in die Polyethylenglykole erfolgt leicht durch Reaktion mit verschiedenartigen Phosphorylierungsreagenzien bei Raum- oder erhöhten Temperaturen.
Siehe **Houben-Weyl**, Bd.XII/2, S.131 ff. (1964),Bd.E2, XII/2, S.300 ff. (**1982**)

Analog erfolgt die Herstellung von phosphatgruppenhaltigen Kohlehydraten, auch von phosphatgruppenhaltigen Polysaccharid-carbonsäuren (siehe US-A-2970141).

Bei der Herstellung von ω-Oxoalkoxy-polyethylenglykol-phosphaten geht man zweckmäßig von den Dimethylacetalen entsprechender Alkohole aus, um die Oxogruppe bei der Ethoxylierung und bei der Einführung der Phosphat- oder Phosphonatgruppe zu schützen. Die Acetale können durch saure Hydrolyse mit Säuren oder durch schonende Spaltung mit Ionenaustauschern freigesetzt werden.
Siehe **Houben-Weyl**, Bd.VI/3, S.203-293 (1964)

Die Herstellung der phosphonatgruppenhaltigen Polyethylenglykole kann nach vielfältigen Methoden erreicht werden.
Siehe **G. M. Kosolapoff**, **L. Maier**, Organic Phosphorous Compounds. Wiley Inters., New York, 1972-1976, Vol.7, S.1-486 (1976),
**Houben-Weyl**, Bd. XII/1, S.338-619 (1963),
**Houben-Weyl**, Bd. E2, S.300-486 (1982),
und insbesondere DE-A-3407565, DE-A-2424453 und DE-A-3203309.

Die Herstellung der silikatgruppenhaltigen organischen Substanzen erfolgt z.B. durch Reaktion von lithium- oder natriumorganischen Verbindungen der allgemeinen Formel
X - R - Li bzw. X - R - Na mit Tetraalkoxysilanen oder Trialkoxychlorsilanen (siehe **Houben-Weyl**, Bd. XIII/5, S.180 ff. (1980)). Eine weitere einfache Möglichkeit zur Herstellung silikatgruppenhaltiger organischer Verbindungen ist die Umsetzung der X - R - (p-Toluolsulfonate) mit z.B. 2-Mercaptopropyl-triethoxysilan, 3-Aminopropyl-triethoxysilan im alkalischen Medium (siehe K.Nilsson, K.Mosbach;Eur.J.Biochem. 112, 397-409,1980).

Die Herstellung der mercaptogruppenhaltigen organischen Substanzen erfolgt z.B aus den X - R - Halogen-Verbindungen durch Umsetzung mit Thioharnstoff und nachfolgender alkalischer Hydrolyse zu den entsprechenden Mercaptoverbindungen (siehe Houben-Weyl, Bd.IX, S.3 ff. (1955),Bd.E2, XI E, S.32 ff.(1985)).

Die Herstellung der polycarbonsäuregruppenhaltigen Kohlehydrate erfolgt durch Oxidation der Kohlehydrate z.B. mit Kaliumpermanganat, Eisen(II)-salzen/Wasserstoffperoxid, Perjodsäure (siehe A.H.Haines, Hrsg., Methods for the Oxidation of Organic Compounds, Academic Press, London, 1988).

Die Herstellung der silikatgruppenhaltigen anorganischen Kondensationsprodukte erfolgt durch einfaches Mischen von Natriumsilikatlösung mit z.B. Natriumaluminat.

Die Herstellung der polysulfatgruppenhaltigen Kohlehydrate erfolgt z.B. durch Chlorsulfonierung von Kohlehydraten.

Das Hauptanwendungsgebiet der erfindungsgemäßen superparamagnetischen Teilchen liegt auf dem Gebiet des magnetischen drug targeting. Aufgrund der sehr hohen Anteiles an Magnetmaterial ( 90 bis 98 Gew.-%) lassen sich schon kleine Magnetteilchen sehr gut und sehr schnell in bestimmte Regionen des Körpers mit Hilfe von elektromagnetischen oder Permanentmagnet-Feldern konzentrieren. Bei Kopplung von pharmakologisch wirksamen Substanzen an die superparamagnetischen Teilchen, kann deren Konzentration am Wirkungsort drastisch erhöht werden. Dieser Umstand hat für die Krebstherapie besondere Bedeutung, da die zur Chemotherapie von Tumoren eingesetzten Substanzen sehr starke Nebenwirkungen auf den gesamten Organismus ausüben und bei einer Anreicherung am Wirkungsort der übrige Körper weniger stark mit Zytostatika belastet wird.

Die superparamagnetischen Teilchen können durch Kopplung an Viren, Zellen und deren Oberflächenmolekülen zur Immunaktivierung im Körper eingesetzt werden, wobei die Einwirkung von Magnetfeldern die Immunaktivierung unterstützt.

Die reaktiven superparamagnetischen Teilchen können auch zur in vitro Diagnostik eingesetzt werden, wenn auf der Oberfläche der Teilchen die entsprechenden diagnostischen Substanzen chemisch gebunden werden. Aufgrund der starken magnetischen Wechselwirkung mit Magnetfeldern, lassen sich auch sehr kleine superparamagnetische Teilchen nach erfolgter diagnostischer Reaktion leicht aus dem Reaktionsgemisch wieder abtrennen.

Die superparamagnetischen Teilchen können auch als Kontrastmittel für Kernspin-Diagnostik eingesetzt werden.

Die Konzentration der einzusetzenden superparamagnetischen Teilchen ist beim magnetischen drug targeting abhängig von der Teilchengröße, von der Zusammensetzung der Stabilisatorsubstanzen, von der magnetischen Feldstärke am Wirkungsort und wie groß die Entfernung von der Injektionsstelle zum Wirkungsort ist. Um eine Nekrose eines Tumores bei Nacktmäusen zu erreichen, ist eine Menge an Injektionsflüssigkeit von ca. 0,01 bis 0,2 Vol-% des Blutvolumens notwendig, wobei die magnetischen Sättigungsinduktion bei ca. 5 mT liegt.

Die Mengen an superparamagnetischen Teilchen liegen bei der Anwendung als Kontrastmittel für die MRI bei ca.0,001 Vol-% des Blutvolumens, wenn die magnetische Sättigungsinduktion bei ca. 5 mT liegt.

An Beispielen sollen die Herstellung der erfindungsgemäßen superparamagnetischen Teilchen erläutert werden.

### Beispiel 1

Eisen(III)-chlorid (270 g) und Eisen(II)-chlorid(119 g) werden in 1 l dest. Wasser gelöst. Durch Zugabe von Ammoniakwasser wird unter Rühren der pH-Wert der Lösung auf 9,6 eingestellt. Nach erfolgter Fällung wird die Dispersion mit Salzsäure auf den pH 6,0 eingestellt und die Dispersion auf 100°C erwärmt. Nach dem Abkühlen wird der Niederschlag mit dest. Wasser gewaschen, bis die elektrische Leitfähigkeit < 10µS/cm beträgt.
Die gebildeten superparamagnetischen Teilchen bestehen aus Fe₃O₄. Sie können stabilisiert werden.

### Beispiel 2

Eisen(III)-chlorid (270 g) und Eisen(II)-sulfat (153 g) werden in 1 l dest. Wasser gelöst. Durch Zugabe von Ammoniakwasser wird unter Rühren der pH-Wert der Lösung auf 9,0 eingestellt. Nach erfolgter Fällung wird die Dispersion unter Rühren mit Salzsäure auf pH 5,0 eingestellt und mit 30%-iger Wasserstoffperoxidlösung (22 ml) versetzt und 30 min auf 80°C erwärmt. Nach dem Abkühlen der Dispersion wird der Niederschlag gewaschen bis die elektrische Leitfähigkeit < 10 µS/cm beträgt. Die entstandenen superparamagnetischen Teilchen aus γ-Fe2O3 und können stabilisiert werden.

### Beispiel 3

Eisen (III)-chlorid (270 g) und Zinkchlorid (82 g) werden in 1 l dest. Wasser gelöst. Durch Zugabe von Natronlauge wird unter Rühren ein pH-Wert von 8,5 eingestellt.Nach erfolgter Fällung wird die Dispersion unter Rühren mit Salzsäure auf den pH-Wert von 4,0 eingestellt und auf 110°C im Autoklaven erwärmt. Nach dem Abkühlen der Dispersion wird der Niederschlag gewaschen, bis das Filtrat eine elektrische Leitfähigkeit von < 10 = µS/cm besitzt. Das entstehende Zink-Ferrit kann stabilisiert werden.

Die Stabilisierung der superparamagnetischen Teilchen erfolgt durch Mischen einer wäßrigen oder niedrigsiedende polare Lösungsmittel enthaltenden Stabilisatorlösung mit den Magnetteilchen bei Raumtemperatur. Die Stabilisatorlösung kann dabei, je nach den gewünschten Eigenschaften, aus reinen Stabilisatorsubstanzen oder aus Mischungen von Stabilisatorsubstanzen bestehen. Zur Beschleunigung der Dispergierung und Stabilisierung kann die Dispersion gerührt oder mit Ultraschall behandelt werden. Kommen niedrigsiedende organische Lösungsmittel zur Anwendung, werden diese zur Entfernung nach der Stabilisierung durch Vakuumverdampfung oder Dialyse entfernt.

An einigen Beispielen soll die Stabilisierung der superparamagnetischen Teilchen nachfolgend erläutert werden.

### Beispiel 4

Die gesamte Menge des Magnetit-Niederschlages von Beispiel 1 wird in eine Lösung von 50 g Mono[ω-Methoxy-polyethylenglykol]-phosphat (Molekulargewicht ca.1000) in 500 ml dest. Wasser gegeben und 5 min gerührt. Die entstehende Dispersion wird 30 min auf einen Permanentmagneten mit einer magnetischen Flußdichte von 0,1 T sedimentiert und der Überstand von magnetischer Flüssigkeit abgesaugt. Das Sediment auf dem Magnetfeld enthält die superparamagnetischen Teilchen. Durch mehrmaliges Waschen mit dest. Wasser und erneuter Sedimentation im Magnetfeld können die superparamagnetischen Teilchen rein und in enger Teilchengrößenverteilung erhalten werden. Die superparamagnetischen Teilchen haben einen mittleren Teilchendurchmesser von 120 nm.

Diese superparamagnetischen Teilchen sind sehr gut für die magnetische Anreicherung in Tumoren geeignet. Hier können sie durch magnetomechanische Immunstimulierung, oder zusätzlich durch Hyperthermie, d.h. durch Einstrahlung elektromagnetischer Strahlung und Erwärmung des Tumors, den Tumor zerstören. Die superparamagnetischen Teilchen sind auch als orales oder i.v. Kontrastmittel für die MRI anwendbar.

### Beispiel 5

Die gesamte Menge des Zink-Ferrit-Niederschlages von Beispiel 3 wird in eine Lösung von 50 g Di[ω-Methoxy-polyethylenglykol]-phosphat (Molekulargewicht ca. 1500) in 500 ml dest. Wasser gegeben und 5 min mit Ultraschall von 100 W Leistung dispergiert. Die entstehenden superparamagnetischen Teilchen haben einen Durchmesser von 310 nm.Die Dispersion wird gegen dest. Wasser dialysiert und durch ein 0,45-µm-Filter filtriert. Das entstehende Produkt ist als orales Kontrastmittel für das MRI anwendbar.

### Beispiel 6

Die gesamte Menge an γ-Fe2O3 Teilchen von Beispiel 2 wird in eine Lösung von 20 g Mono-[ω-Oxoethoxy-polyethylenglykol]-phosphat (Molekulargewicht ca. 1800), 15 g Mono- und 15 g Di-[ω-Methoxy-polyethylenglykol]-phosphat (Molekulargewicht ca. 1000) in 500 ml dest. Wasser gegeben und 5 min mit einem Ultraschalldispergator (100 W Leistung) dispergiert. Die entstehende Dispersion wird mit einem 50 kD-Filter gegen dest. Wasser dialysiert, um überschüssige Stabilisatorsubstanzen zu entfernen.
Die Abtrennung der nicht oder nur schwach agglomerierten superparamagnetischen Eindomänenteilchen, die eine stabile magnetische Flüssigkeit bilden, erfolgt durch eine magnetische Sedimentation wie im Beispiel 4 beschrieben. Die superparamagnetischen Teilchen haben einen mittleren Teilchendurchmesser von 180 nm.
Die nach Beispiel 6 hergestellten superparamagnetischen Teilchen sind für viele Kopplungsreaktionen verwendbar, bei denen die Reaktivität der Aldehydgruppe Anwendung finden kann. So z.B. für die Kopplung von aminogruppenhaltigen pharmakologisch wirksamen Substanzen, wie Streptokinase oder Plasminogen-Streptokinase-Aktivator-Komplex.

### Beispiel 7

10 ml der Dispersion von Beispiel 6, mit einer magnetischen Sättigungsinduktion von 5 mT, werden mit 30 mg Anistreplase gemischt und 20 min bei Raumtemperatur stehengelassen. Das entstehende Produkt ist für ein magnetisches drug targeting zur Auflösung von Blutgerinnseln einsetzbar.

Die nach Beispiel 6 hergestellten reaktiven superparamagnetischen Teilchen sind auch für die Herstellung von Substanzen für das magnetische drug targeting in der Tumortherapie geeignet, wie an Beispiel 8 erläutert werden soll.

### Beispiel 8

10 ml der Dispersion von Beispiel 6, mit einer magnetischen Sättigungsinduktion von 5 mT, werden mit 10 ml einer 10-gew.-%-igen MITOMYCIN C Lösung gemischt und 30 min bei Raumtemperatur geschüttelt. Das entstehende Produkt ist für ein magnetisches drug targeting gegen Leukämie anwendbar.

### Beispiel 9

10 ml der Dispersion von Beispiel 6, mit einer magnetischen Sättigungsinduktion von 5 mT, werden mit 10 mg EPIRUBICIN-Hydrochlorid gemischt und 20 min bei Raumtemperatur geschüttelt. Das entstehende Produkt ist für ein magnetisches drug targeting in der Tumortherapie anwendbar.

Erfindungsgemäß können auf der Oberfläche der superparamagnetischen Teilchen auch direkt phosphat- oder phosphonatgruppenhaltige Arzneimittel chemisch gebunden werden, indem die 0,2-bis 0,3-fache Menge der zuzusetzenden Stabilisatormenge in Form des Arzneimittels zum unstabilisierten Niederschlag zugegeben wird und nach 5 min Rühren die entsprechende restliche Menge des Stabilisators, unter weiterem Rühren, zugesetzt wird.

### Beispiel 10

Die gesamte Menge des Magnetit-Niederschlages von Beispiel 1 wird mit einer Lösung von 10 g ESTRAMUSTIN in 250 ml dest. Wasser gemischt und 5 min gerührt. Anschließend wird 40 g Di-[ω-Methoxy-polyethylenglykol]-phosphat (Molekulargewicht ca. 750) in 250 ml dest. Wasser gelöst, zur Mischung gegeben und 5 min gerührt. Die entstandene Dispersion wird 30 min auf einem Permanentmagneten mit einer magnetischen Flußdichte von 0,1 mT sedimentiert und der Überstand von magnetischer Flüssigkeit abgesaugt. Das Sediment auf dem Magneten enthält die superparamagnetischen Teilchen und wird durch ein 0,45-µm-Filter filtriert. Das entstehende Produkt ist für ein magnetisches drug targeting des Prostatakarzinoms anwendbar.

### Beispiel 11

Die gesamte Menge des Magnetitniederschlages von Beispiel 1 wird in eine Lösung eines Gemisches von 40 g Mono-[ω-Methoxypolyethylenglykol]-phosphat, -diphosphat und -polyphosphat, mit dem Mischungsverhältnis von ungefähr 8:1:1 und einem mittleren Molekulargewicht von ca. 750, in 500 ml dest. Wasser gegeben und 5 min gerührt. Die entstehende Dispersion wird 30 min auf einen Permanentmagneten mit einer magnetischen Flußdichte von 0,1 T sedimentiert und der Überstand von magnetischer Flüssigkeit abgesaugt. Das Sediment auf dem Magnetfeld enthält die superparamagnetischen Teilchen. Durch mehrmaliges Waschen mit dest. Wasser und erneuter Sedimentation im Magnetfeld können die superparamagnetischen Teilchen rein und in enger Teilchengrößenverteilung erhalten werden. Die superparamagnetischen Teilchen haben einen mittleren Teilchendurchmesser von 120 nm.

Diese superparamagnetischen Teilchen sind sehr gut für die magnetische Anreicherung in Tumoren geeignet. Hier können sie durch magnetomechanische Immunstimulierung, oder zusätzlich durch Hyperthermie, d.h. durch Einstrahlung elektromagnetischer Strahlung und Erwärmung des Tumors, den Tumor zerstören. Die superparamagnetischen Teilchen sind auch als orales oder i.v. Kontrastmittel für die MRI anwendbar.

Durch Anwendung von Stabilisatorgemischen können die superparamagnetischen Teilchen bei bestimmten, ausgewählten pH-Werten zur Aggregation gebracht werden. So kann die Ausfällung der superparamagnetischen Teilchen in alkalischen Tumoren, zusätzlich zur Anreicherung durch magnetisches drug targeting, zu einer magnetomechanischen Schädigung der Tumoren führen, wenn zum Beispiel ein Gemisch aus Cocarboxylase, Mono- und Di-[ω-Methoxypolyethylenglykol]-phosphat als Stabilisatoren eingesetzt wird.

### Beispiel 12

Die gesamte Menge des Magnetitniederschlages von Beispiel 1 wird in eine Lösung von 15 g Mono-, 15 g Di-[Methoxy-polyethylenglykol]-phosphat (Molekulargewicht ca. 2000) und 12 g Cocarboxylase in 500 ml dest. Wasser gegeben und 5 min mit Ultraschall dispergiert.Die entstehende Dispersion wird 30 min auf einen Permanentmagneten mit einer magnetischen Flußdichte von 0,1 T sedimentiert und der Überstand von magnetischer Flüssigkeit abgesaugt. Das Sediment auf dem Magnetfeld enthält die superparamagnetischen Teilchen. Durch mehrmaliges Waschen mit dest. Wasser und erneuter Sedimentation im Magnetfeld können die superparamagnetischen Teilchen rein und in enger Teilchengrößenverteilung erhalten werden. Die superparamagnetischen Teilchen haben einen mittleren Teilchendurchmesser von 120 nm.

Diese superparamagnetischen Teilchen sind sehr gut für die magnetische Anreicherung in Tumoren geeignet. Hier können sie durch magnetomechanische Immunstimulierung, oder zusätzlich durch Hyperthermie, d.h. durch Einstrahlung elektromagnetischer Strahlung und Erwärmung des Tumors, den Tumor zerstören. Magnetfelder können die Wirksamkeit der Immunstimulierung erhöhen.

Die erfindungsgemäßen neuen diphosphat- und polyphosphathaltigen Stabilisatorsubstanzen können allein, d.h. auch ohne Kopplung an die superparamagnetischen Aggregate, bei systemischer Anwendung im Körper zur Tumorschädigung eingesetzt werden. Dazu verwendet man eine Mischung aus einer oder mehreren der oben beschriebenen Stabilisatorsubstanzen und einem pharmakologisch annehmbaren Träger, wie z.B. physiologische Kochsalzlösung.

### Beispiel 13

4 g Mono-[ω-Methoxy-polyethylenglykol]-diphosphat (Molekulargewicht ca. 1000) werden in 100 ml physiologische Kochsalzlösung gelöst und durch ein 0,2 µm -Filter steril filtriert. Die entstehende Flüssigkeit ist für den Einsatz zur Tumorschädigung geeignet.

### Beispiel 14

Die gesamte Menge des Magnetit-Niederschlages von Beispiel 1 wird in eine Lösung von 50 g einer 40 %-igen Natriumsilikatlösung in 500 ml dest. Wasser gegeben und 5 min gerührt. Die entstehende Dispersion wird 30 min auf einem Permanentmagneten mit einer magnetischen Flußdichte von 0,1 T sedimentiert und der Überstand von magnetischer Flüssigkeit abgesaugt. Das Sediment auf dem Magnetfeld enthält die superparamagnetischen Teilchen. Durch mehrmaliges Waschen mit dest. Wasser und erneuter Sedimentation im Magnetfeld können die superparamagnetischen Teilchen rein und in enger Teilchengrößenverteilung erhalten werden. Die superparamagnetischen Teilchen haben einen mittleren Teilchendurchmesser von 120 nm.

Diese superparamagnetischen Teilchen sind sehr gut für die magnetische Anreicherung in Tumoren geeignet. Hier können sie durch magnetomechanische Immunstimulierung oder zusätzlich durch Hyperthermie, d.h. durch Einstrahlung elektromagnetischer Strahlung und Erwärmung des Tumors, den Tumor zerstören. Die superparamagnetischen Teilchen sind auch als orales oder i.v. Kontrastmittel für die MRI anwendbar.

### Beispiel 15

Die gesamte Menge des γ-Fe₂O₃ -Niederschlages von Beispiel 2 wird in eine Lösung von 20g 3-Mercaptopropyl-trimethoxysilan in 500 ml dest. Wasser gegeben und 5 min mit Ultraschall von 100 W Leistung dispergiert.

Die entstehenden superparamagnetischen Teilchen haben einen Durchmesser von 310 nm. Die Dispersion wird gegen dest. Wasser dialysiert und durch ein 0,45 µm-Filter filtriert. Das entstehende Produkt ist für die Kopplung von monoklonalen Antikörpern anwendbar.

### Beispiel 16

Die gesamte Menge des Magnetit-Niederschlages von Beispiel 1 wird in eine Lösung von 40g ω-Methoxy-polyethylenglykol-trimethoxysilan (Molekulargewicht 1000) in 500 ml Wasser gegeben und 10 min mit einem Ultraschalldispergator (100W Leistung), unter Erwärmen auf 70 °C, dispergiert. Die Abtrennung der nicht oder nur schwach agglomerierten superparamagnetischen Eindomänenteilchen, die eine stabile magnetische Flüssigkeit bilden, erfolgt durch eine magnetische Sedimentation wie im Beispiel 4 beschrieben.

An die Polyalkylenglykolgruppen-haltigen superparamagnetischen Teilchen lassen sich auch diamagnetische Polyalkylengruppen-haltige pharmakologisch wirksame Substanzen adsorptiv binden, wobei letztere Substanzen beim magnetischen drug targeting, unter der Einwirkung eines inhomogenen Magnetfeldes, wieder von der Oberfläche der superparamagnetischen Teilchen desorbiert werden. Erfolgt eine adsorptive Bindung von z.B. Doxorubucin-Monopolyethylenglykol-phosphat an Teilchen nach Beispiel 5, so kann diese Produkt für ein magnetisches drug targeting von Zytostatika in der Tumortherapie eingesetzt werden.

### Beispiel 17

Die gesamte Menge des Magnetit-Niederschlages von Beispiel 1 wird in eine Lösung von 45g ω-Oxoethoxy-polyethylenglykolsilantriol (Molekulargewicht ca.1800) in 500 ml dest. Wasser gegeben und 5 min mit einem Ultraschalldispergator (100 W Leistung) dispergiert. Die entstehende Dispersion wird mit einem 50-kD-Filter gegen dest. Wasser dialysiert, um überschüssige Stabilisatorsubstanzen zu entfernen.

Die Abtrennung der nicht oder nur schwach agglomerierten superparamagnetischen Eindomänenteilchen, die eine stabile magnetische Flüssigkeit bilden, erfolgt durch eine magnetische Sedimentation wie im Beispiel 4 beschrieben. Die superparamagnetischen Teilchen haben einen mittleren Teilchendurchmesser von 180 nm.

Die nach diesem Beispiel hergestellten superparamagnetischen Teilchen sind für viele Kopplungsreaktionen verwendbar, bei denen die Reaktivität der Aldehydgruppe Anwendung finden kann. So z.B. für die Kopplung von aminogruppenhaltigen pharmakologisch wirksamen Substanzen, wie Streptokinase oder Plasminogen-Streptokinase-Aktivator-Komplex.

### Beispiel 18

10 ml der Dispersion von Beispiel 17, mit einer magnetischen Sättigungsinduktion von 5 mT, werden mit 30 mg Anistreplase gemischt und 20 min bei Raumtemperatur stehengelassen. Das entstehende Produkt ist für ein magnetisches drug targeting zur Auflösung von Blutgerinnseln geeignet. Beispiel 19:

10 ml der Dispersion von Beispiel 17, mit einer magnetischen Sättigungsinduktion von 5 mT, werden mit 10ml einer 10 mg DOXORUBICIN enthaltenden Lösung gemischt und 30 min bei Raumtemperatur geschüttelt. Das entstehende Produkt ist für ein magnetisches drug targeting in der Tumortherapie anwendbar.

Diese superparamagnetischen Teilchen sind für die magnetische Anreicherung in Tumoren geeignet und das tumorschädigende Zytostatikum kann in erhöhter Konzentration auf den Tumor einwirken. Zusätzlich kann eine Tumorschädigung durch Hyperthermie, d.h. durch Einstrahlung elektromagnetischer Strahlung und Erwärmung des Tumors vorgenommen werden.

Magnetfelder können die Wirksamkeit der Immunstimulierung erhöhen.

### Beispiel 20

Die gesamte Menge des Magnetit-Niederschlages von Beispiel 1 wird in eine Lösung von 25 g einer Mischung aus 70 % Natriumsilikat und 30 % Natriumaluminat in 500 ml Wasser gegeben und 10 min mit einem Ultraschalldispergator (100W Leistung), unter Erwärmen auf 70 °C, dispergiert. Nach dem Neutralisieren der Dispersion mit verd. Salzsäure auf einen pH-Wert von 7, erfolgt die Abtrennung der nicht oder nur schwach agglomerierten superparamagnetischen Eindomänenteilchen, die eine stabile magnetische Flüssigkeit bilden, durch eine magnetische Sedimentation wie im Beispiel 4 beschrieben. Diese superparamagnetischen Teilchen sind als orales Kontrastmittel für den gastro-intestinalen Bereich anwendbar.

### Beispiel 21

Die gesamte Menge des Magnetit-Niederschlages von Beispiel 1 wird in eine Lösung von 20 g Natriumsalzes des Dextransulfates (Molekulargewicht 40.000) in 500 ml Wasser gegeben und 10 min mit einem Ultraschalldispergator (100 W Leistung), unter Erwärmen auf 70 °C, dispergiert und die Abtrennung der nicht oder nur schwach agglomerierten superparamagnetischen Eindomänenteilchen, die eine stabile magnetische Flüssigkeit bilden, erfolgt durch eine magnetische Sedimentation wie im Beispiel 4 beschrieben. Diese superparamagnetischen Teilchen sind als orales Kontrastmittel für den gastro-intestinalen Bereich anwendbar.

Die superparamagnetischen Teilchen können auch, gekoppelt mit Tensiden, Zellmembranen zerstören und so eine Zellschädigung in vitro und in vivo hervorrufen. Als Tenside können z.B. Nonylphenol-polyethylenglykol-phosphat, Alkyl- oder Alkylaryl-polyethylenglykol-sulfate (Zahl der Ethylenoxidgruppen zwischen 5 und 40) Anwendung finden.

Um eine magnetomechanische Immunstimulierung im Körper einzuleiten, werden magnetisierbare Leukozyten parenteral in den Körper gegeben und mit Hilfe von Magnetfeldern am Wirkungsort angereichert. Durch die magnetomechanischen Kräfte werden die magnetisierten Leukozyten, und hier vor allen Dingen die neutrophilen Granulozyten und Makrophagen, in den Hochendothel-Venolen angereichert, beschleunigt das Endothel durchdringen und in das Gewebe eindringen. Die Anreicherung der magnetisierbaren Leukozyten, z.B. im Tumorgewebe, kann zu dessen immunologischen Schädigung führen. Vor allen Dingen dann, wenn eine magneto-mechanische Schädigung der Tumormembran durch die superparamagnetischen Teilchen eintritt und die Tumormembranbruchstücke von den benachbarten neutrophilen Granulozyten und Makrophagen phagozytiert werden. Die Ausbildung von Tumor-Antikörpern kann dann auch zur Schädigung von nicht behandelten Tumoren führen. Magnetfelder können die Wirksamkeit der Immunstimulierung erhöhen.

Zur Herstellung eines magnetisierbaren Leukozytenkonzentrates wird ein Leukozytenkonzentrat mit reaktiven superparamagnetischen Teilchen gemischt und bei Raumtemperatur oder bei 37°C zur Reaktion gebracht.

### Beispiel 22

100 ml Leukozytenkonzentrat werden mit 10 ml der Dispersion von Beispiel 6, mit einer magnetischen Sättigungsinduktion von 5 mT, gemischt und 20 min bei 37°C stehengelassen. Das entstehende Produkt ist für ein magnetisches drug targeting zur Tumorschädigung einsetzbar.

Eine magneto-mechanische Immunstimulierung kann z.B. auch mit magnetisierbaren Viren, Bakterien, Pilzen, Tumorzellen oder deren magnetisierbaren Oberflächenmolekülen erfolgen. Die magnetisierbaren Viren, Zellen oder deren Oberflächenmoleküle können parenteral in den Körper gegeben werden, da sie dort vom retikuloendothelialem System erkannt und von den Makrophagen und den neutrophilen Granulozyten gebunden und phagozytiert werden. Als Folge der Phagozytose werden Teile der Oberflächenmoleküle als Komplex mit ebenfalls wiederverwendeten Glycoproteinen aus dem Haupt-Histokompatibilitätskomplex an die Oberfläche der Makrophagen zurücktransportiert, wo sie von den T-Lymphozyten des Immunsystem überprüft, als Antigen erkannt und die entsprechende Immunantwort gegen die Oberflächenmoleküle aktiviert wird. Somit erfolgt eine Immunaktivierung des Körpers gegen die entsprechenden Viren und Zellen. Magnetfelder können die Wirksamkeit der Immunstimulierung erhöhen.

So kann eine Immunisierung und Behandlung von schwer therapierbaren Viren-, Bakterien- oder Pilzerkrankungen ermöglicht werden.

Die Herstellung magnetisierbarer Viren oder Zellen erfolgt durch einfaches Mischen derselben mit reaktiven superparamagnetischen Teilchen, z.B. nach Beispiel 6. Durch Zugabe eines pharmakologisch annehmbaren Trägers zur magnetisierbaren Mischung erhält man eine pharmakologisch wirksame Zubereitung zur Immunsteigerung gegen Viren und Zellen.

Die Herstellung magnetisierbarer Oberflächenmoleküle von Viren, Bakterien oder Tumorzellen erfolgt durch einfaches Mischen dieser biologischen Teilchen mit den reaktiven superparamagnetischen Teilchen und nachfolgender Blockierung der restlichen reaktiven Gruppen durch geeignete, physiologisch verträgliche Substanzen. So erfolgt z.B. das Blockieren der Überschüssigen Aldehydgruppen der Stabilisatorsubstanz nach Beispiel 6, durch Zugabe einer 0,5 molare Äthanolamin-hydrochloridlösung (pH 8,5) zur Teilchendispersion. Nach der Zerstörung der magnetisierten biologischen Teilchen mit Hilfe bekannter Aufschlußverfahren, wie Druckaufschluß, mechanisches Zermahlen, osmotische Schockbehandlung, werden die magnetisierten Oberflächenmoleküle mit Hilfe eines Magnetfeldes aus der Aufschlußdispersion entfernt. Die Reinigung der magnetisierbaren Oberflächenmoleküle erfolgt durch mehrmaliges Waschen und Abtrennen im Magnetfeld. Durch Zugabe eines pharmakologisch annehmbaren Trägers zu den gereinigten magnetisierbaren Oberflächenmolekülen erhält man eine pharmakologisch wirksame Zubereitung zur Immunsteigerung gegen Viren und Zellen.

Da die Magnetisierung der Viren, Bakterien, Pilze, Tumorzellen und deren Oberflächenmoleküle in vitro erfolgt, kann auch eine patientenspezifische Immunbehandlung erfolgen. Voraussetzung dafür ist, daß eine Isolierung oder Anreicherung der entsprechenden Viren und Zellen aus dem Körper des Patienten erfolgt.

Die superparamagnetischen Teilchen können auch, gekoppelt mit Tensiden, Zellmembranen zerstören und so eine Zellschädigung in vitro und in vivo hervorrufen. Als Tenside können z.B. Nonylphenol-polyethylenglykol-phosphat, Alkyl- oder Alkylarylpolyethylenglykol-sulfate (Zahl der Ethylenoxidgruppen zwischen 5 und 40) Anwendung finden.

Das Hauptanwendungsgebiet der erfindungsgemäßen superparamagnetischen Teilchen liegt auf dem Gebiet des magnetischen drug targeting. Aufgrund der sehr hohen Anteiles an Magnetmaterial ( 90 bis 98 Gew.-%) lassen sich schon kleine Magnetteilchen sehr gut und sehr schnell in bestimmte Regionen des Körpers mit Hilfe von elektromagnetischen oder Permanentmagnet-Feldern konzentrieren. Bei Kopplung von pharmakologisch wirksamen Substanzen an die superparamagnetischen Teilchen, kann deren Konzentration am Wirkungsort drastisch erhöht werden. Dieser Umstand hat für die Krebstherapie besondere Bedeutung, da die zur Chemotherapie von Tumoren eingesetzten Substanzen sehr starke Nebenwirkungen auf den gesamten Organismus ausüben und bei einer Anreicherung am Wirkungsort der übrige Körper weniger stark mit Zytostatika belastet wird.

Die superparamagnetischen Teilchen können durch Kopplung an Viren, Zellen und deren Oberflächenmolekülen zur Immunaktivierung im Körper eingesetzt werden, wobei die Einwirkung von Magnetfeldern die Immunaktivierung unterstützt.

Die reaktiven superparamagnetischen Teilchen können auch zur in vitro Diagnostik eingesetzt werden, wenn auf der Oberfläche der Teilchen die entsprechenden diagnostischen Substanzen chemisch gebunden werden. Aufgrund der starken magnetischen Wechselwirkung mit Magnetfeldern, lassen sich auch sehr kleine superparamagnetische Teilchen nach erfolgter diagnostischer Reaktion leicht aus dem Reaktionsgemisch wieder abtrennen.

Die superparamagnetischen Teilchen können auch als Kontrastmittel für Kernspin-Diagnostik eingesetzt werden.

Die Mengen an superparamagnetischen Teilchen liegen bei der Anwendung als Kontrastmittel für die MRI bei ca.0,001 Vol-% des Blutvolumens, wenn die magnetische Sättigungsinduktion bei ca. 5 mT liegt.

## Patentansprüche

1. Superparamagnetische Teilchen, bestehend aus Eindomänenteilchen und Stabilisatorsubstanzen, dadurch gekennzeichnet, daß superparamagnetische Eindomänenteilchen aus Eisenoxid-, Eisenmischoxid- oder Eisen mit einer Teilchengröße im Bereich zwischen 3 und 20 Nanometer zu stabilen, abbaubaren, ein definiertes Verhalten im Magnetfeld zeigenden Aggregaten zusammengelagert sind, mit einer Teilchengröße der Aggregate im Bereich zwischen 10 und 1000 Nanometer, wobei die Aggregate auf ihrer Oberfläche eine monomolekulare Schicht aufweisen, bestehend aus Stabilisatorsubstanzen der Gruppe der phosphat-, diphosphat-, carboxylat-, polyphosphat-, thiophosphat-, phosphonat-, thiophosphonat-, sulfat-, sulfonat-, mercapto-, silantriol-, trialkoxysilangruppenhaltigen Polyalkylenglykole, der Kohlehydrate oder der phosphatgruppenhaltigen Nucleotide, deren Oligomere oder deren Polymere, die weitere Bindungsstellen haben können.

2. Superparamagnetische Teilchen nach Anspruch 1, dadurch gekennzeichnet, daß die Teilchengröße der superparamagnetischen Eindomänenteilchen im Bereich von 3 bis 20 nm liegt und die Teilchengröße der superparamagnetischen Aggregate im Bereich von 10 bis 1000 nm.

3. Superparamagnetische Teilchen nach Anspruch 1 oder 2, dadurch gekennzeichnet, daß die superparamagnetischen Eindomänenteilchen aus γ-Fe₂O₃, Fe₃O₄, aus den Eisenmischoxiden der allgemeinen Formel MO·Fe₂O₃, worin M die zweiwertigen Metallionen Fe, Mg, Be, Mn, Zn, Co, Ba, Sr, Cu oder Gemische davon bedeuten, aus den Mischoxiden der allgemeinen Formel mFe₂O₃ · nMe₂O₃, worin Me die dreiwertigen Metallionen Al, Cr, seltene Erdmetalle oder Gemische davon bedeuben; oder Eisen bestehen.

4. Superparamagnetische Teilchen nach einem der Ansprüche 1 bis 3, dadurch gekennzeichnet, daß die Stabilisatorsubstanzen ausgewählt sind unter
(i) den Verbindungen der allgemeinen Formel
X - R - A - B
worin X eine funktionelle Gruppe darstellt, ausgewählt aus der Alkoxy-, Monolkylamino-, Dialkylamino-, Trialkylamino- und Alkylthiogruppe, bei denen die Zahl der Kohlenstoffatome im Alkylteil dieser Gruppen im Bereich von 1 und 4 liegt, oder eine funktionelle Gruppe darstellt, ausgewählt unter der Hydroxyl-, Amin-, Aldehyd-, Dimethylacetal-, Diethylacetal Epoxy-, Thiol-, Carboxy-, 4,6-Dichlortriazin-, Hydroxamsäure-, Isocyanat-, Acylazid-, Anhydrid-, Diazoniumsalz-, Iminocarbonat- und Toluolsulfonatgruppe;
R fehlt oder
R ist ein Polyalkylenglykol, ein mit Wasser mischbarer Polypropylenglykolrest oder ein mit Wasser mischbarer Block-Copolymerisatrest aus Polyethylenglykol (PEG) und Polypropylenglykol (PPG), ausgewählt unter den Block-Copolymerisaten
(PEG)ₐ-(PEG)_{b} , (PEG)ₐ-(PPG)_{b}-(PEG)ₐ, (PPG)_{b}-(PEG)ₐ-(PPG)_{b}
wobei a eine positive ganze Zahl im Bereich von 1 bis 100 und b eine positive ganze Zahl im Bereich von 1 bis 20 darstellt;
n ist eine positive ganze Zahl, ausgewählt für PEG im Bereich von 4 bis 300, für PPG im Bereich von 3 bis 12 und für PEG-PPG-Block-copolymerisat im Bereich von 3 bis 140; oder
R ist ein Kohlehydratrest, ausgewählt aus den Monosacchariden Glucose, Fructose, Ribose, Desoxyribose, Inosit, aus den Oligosacchariden Saccharose, Raffinose, Gentianose, Malecitose, Stachyose, Verbascose, aus den Polysacchariden Stärke, Lichenine, Glykogen, Dextrine, Dextrane, Inuline, Fruktosane, Lävane, Mannane, Galaktane, Xylane, Arabane, Pektine, Makropolysaccharide, Glycoproteide, aus Polyuridenylsäure, Polyglucuronsäure, Polygalacturonsäure, Polymannuronsäure und/oder Alginsäure bestehen;
A fehlt oder
A ist eine Alkyl-, Alkoxy-, Acyl-, Acylamin-, Alkylamingruppe, bei denen die Zahl der Kohlenstoffatome der Alkoxy-, Acyl-, Acylamin-, Alkylgruppe im Bereich von 1 bis 4 liegt;
B ist ein phosphorhaltiger Rest, ausgewählt unter Monophosphat, Diphosphat, Polyphosphat, Phosphonat, Thiophosphat, Thiophosphonat, oder ein carboxylat-, sulfat-, sulfonat-, mercapto-, silantriol-oder trialkoxysilanhaltiger Rest;
(ii) den phosphatgruppenhaltigen Nucleotiden Mono-, Di-, Tri-phosphorsäureester oder Mono-, Di-, Tri-phosphorsäureesterchloriden von Adenosin, Guanosin, Cytidin, Uridin, Thymidin, Desoxyadenosin, Desoxyguanosin, Desoxycytidin, Desoxythymidin, Inosin, Pyrimidin, Cytosin, Uracil, Thymin, Purin, Adenin, Guanin, Methylcytosin, 5-Hydroxymethyl-cytosin, 2-Methyladenin, 1-Methylguanin, Thiamin, Flavin, Riboflavin sowie Pyridoxalphosphat, Pyridoxaminphosphat, Ribonucleinsäure, Ribonucleinsäuresequenzen, Desoxyribonucleinsäuren, Desoxyribonucleinsäuresequenzen;
(iii) den silicatgruppenhaltigen Verbindungen der Orthokieselsäure und deren Kondensationsprodukte; und /oder
(iv) X - R - A - B ist Mercaptopurin, -cytosin, -guanin, -uracil, -thymin, -hypoxanthin, sowie deren Mercapto-nucleoside und deren Mercapto-desoxynucleoside;
(v) X - R - A - B ist ein Polyaminokohlehydrat.

5. Superparamagnetische Teilchen nach einem der Ansprüche 1 bis 4, dadurch gekennzeichnet, daß an die superparamagnetischen Teilchen
(i) eine gewebespezifische Bindungssubstanz aus der Gruppe der Antigene, Antikörper, Ribonucleinsäuren, Desoxyribonucleinsäuren, Ribonucleinsäuresequenzen, Desoxyribonucleinsäuresequenzen, Haptene, Protein A, Protein G, Endotoxin-bindende Proteine, Lectine, Selectine;
(ii) eine pharmakologisch wirksame Substanz aus der Gruppe der Antitumorproteine, Enzyme, Antitumorenzyme, Antibiotika, Pflanzenalkaloide, Alkylierungsreagenzien, Antimetaboliten, Hormone und Hormonantagonisten, Interleukine, Interferone, Wachstumsfaktoren, Tumornekrosefaktoren, Endotoxine, Lymphotoxine, Urokinase, Streptokinase, Plasminogen-Streptokinase-Aktivator-Komplex, Gewebe-Plasminogen-Aktivatoren, Desmodus-Plasminogen-Aktivatoren, Makrophagen-Aktivierungskörper, Antisera, Proteaseninhibitoren und/oder radioakiven Phosphor ³²P enthaltende Stabilisatorsubstanzen, Tenside;
(iii) pharmakologisch wirksame Zellen aus der Gruppe der Organellen, Viren, Mikroben, Algen, Pilze, insbesondere Erythrozyten, Thrombozyten, Granulozyten, Monozyten, Lymphozyten und/oder Langerhans'sche Inseln; (iv) pharmakologisch wirksamer Komplexbildner aus der Gruppe der Polycarbonsäuren, Aminocarboxylsäuren, Porphyrinen, Katecholamine;
(v) phosphat- oder phosphonatgruppenhaltige Arzneimittel; und/oder
(vi) zellfusionvermittelnde Substanzen chemisch gebunden sind.

6. Verfahren zur Herstellung superparamagnetischer Teilchen, bestehend aus Eindomänenteilchen und Stabilisatorsubstanzen, dadurch gekennzeichnet, daß superparamagnetischen Eindomänenteilchen aus Eisenoxid-, Eisenmischoxid- oder Eisen mit einer Teilchengröße im Bereich zwischen 3 und 20 Nanometer durch Fällung aus wäßrigen Eisensalzlösungen mit Alkalilauge oder Ammoniakwasser im pH-Bereich von 8,0 bis 10,0 hergestellt, mit einer Säure auf einen pH-Wert zwischen 3 und 7 eingestellt und bei einer Temperatur im Bereich von 50 bis 120 °C und gegebenenfalls erhöhtem Druck in diesem pH- und Temperaturbereich zur Aggregation gebracht werden zu Aggregaten mit einer Teilchengröße im Bereich zwischen 10 und 1000 Nanometer; die Aggregate in an sich bekannter Weise gereinigt und mit 20 bis 50 Gew.-% Stabilisatorsubstanz versetzt werden, wobei die Stabilisatorsubstanz ausgewählt ist aus der Gruppe der phosphat-, diphosphat-, carboxylat-, polyphosphat-, thiophosphat-, phosphonat-, thiophosphonat-, sulfat-, sulfonat-, mercapto-, silantriol-, trialkoxysilangruppenhaltigen Polyalkylenglykole, der Kohlehydrate oder der phosphatgruppenhaltigen Nucleotide, deren Oligomere oder deren Polymere, die weitere Bindungsstellen haben können; und die erhaltenen stabilen, abbaubaren Aggregate mit definiertem Verhalten im Magnetfeld in an sich bekannter Weise gereinigt und gegebenenfalls noch mit pharmakologisch oder diagnostisch wirksamen Substanzen in an sich bekannter Weise gekoppelt werden.

7. Pharmakologisch wirksame Zubereitung aus einem pharmakologisch annehmbaren Träger und superparamagnetischen Teilchen, bestehend aus superparamagnetischen Eindomänenteilchen aus Eisenoxid-, Eisenmischoxid- oder Eisen mit einer Teilchengröße im Bereich zwischen 3 und 20 Nanometer, die zu stabilen, abbaubaren, ein definiertes Verhalten im Magnetfeld zeigenden Aggregaten zusammengelagert sind, mit einer Teilchengröße der Aggregate im Bereich zwischen 10 und 1000 Nanometer, wobei die Aggregate auf ihrer Oberfläche eine monomolekulare Schicht aufweisen, bestehend aus Stabilisatorsubstanzen der Gruppe der phosphat-, diphosphat-, carboxylat-, polyphosphat-, thiophosphat-, phosphonat-, thio-phosphonat-, sulfat-, sulfonat-, mercapto-, silantriol-, trialkoxysilangruppenhaltigen Polyalkylenglykole, der Kohlehydrate oder der phos-phatgruppenhaltigen Nucleotide, deren Oligomere oder deren Polymere, die weitere Bindungsstellen haben können, gegebenenfalls in Verbindung mit einer gewebespezifischen Bindungssubstanz, einer pharmakologisch wirksamen Substanz, einer pharmakologisch wirksamen Zelle, einem pharmakologisch wirksamen Komplexbildner, einem Arzneimittel oder einer zellfusionsvermittelnden Substanz der in Anspruch 5 genannten Gruppen.

8. Verwendung der Zusammensetzung nach Anspruch 7 zur Herstellung einer pharmakologisch wirksamen Zubereitung zur Tumorschädigung und Immunsteigerung, die wahlweise unter Einwirkung von Magnetfeldern eingesetzt wird.

## Claims

1. Superparamagnetic particles, consisting of single-domain particles and stabilizer substances, characterized in that superparamagnetic single-domain particles consisting of iron oxide, iron mixed oxide or iron with a particle size in the range between 3 and 20 nanometres are combined to form stable, decomposable aggregates exhibiting defined behaviour in the magnetic field, with a particle size of the aggregates in the range between 10 and 1000 nanometres, wherein the aggregates have a monomolecular layer on their surface, consisting of stabilizer substances from the group comprising the phosphate, diphosphate, carboxylate, polyphosphate, thiophosphate, phosphonate, thiophosphonate, sulphate, sulphonate, mercapto, silanetriol, trialkoxysilane group-containing polyalkylene glycols, the carbohydrates or the phosphate group-containing nucleotides, their oligomers or their polymers, which can have further binding sites.

2. Superparamagnetic particles according to Claim 1, characterized in that the particle size of the superparamagnetic single-domain particles lies in the range of 3 to 20 nm and the particle size of the superparamagnetic aggregates in the range of 10 to 1000 nm.

3. Superparamagnetic particles according to Claim 1 or 2, characterized in that the superparamagnetic single-domain particles consist of γ-Fe₂0₃, Fe₃0₄, of the iron mixed oxides corresponding to the general formula MO·Fe₂0₃, wherein M represents the divalent metal ions Fe, Mg, Be, Mn, Zn, Co, Ba, Sr, Cu or mixtures thereof, of mixed oxides corresponding to the general formula mFe₂0₃ · nMe₂0₃, wherein Me represents the trivalent metal ions Al, Cr, rare earth metals or mixtures thereof, or of iron.

4. Superparamagnetic particles according to one of Claims 1 to 3, characterized in that the stablizer substances are selected from among
(i) the compounds corresponding to the general formula
X - R - A - B
wherein
X represents a functional group selected from the alkoxy-, monoalkylamino, dialkylamino, trialkylamino and alkylthio group in which the number of carbon atoms in the alkyl part of these groups lies in the range of 1 and 4, or a functional group selected from the hydroxyl, amine, aldehyde, dimethylacetal, diethylacetal, epoxy, thiol, carboxy, 4,6-dichlorotriazine, hydroxamic acid, isocyanate, acylazide, anhydride, diazonium salt, iminocarbonate and toluene sulphonate group;
R is absent or
R is a polyalkylene glycol, a water-miscible polypropylene glycol radical or a water-miscible block copolymer radical of polyethylene glycol (PEG) and polypropylene glycol (PPG), selected from the block copolymers
(PEG)ₐ-(PEG)_{b}, (PEG)ₐ-(PPG)_{b}-(PEG)ₐ, (PPG)_{b}-(PEG)ₐ-(PPG)_{b}
wherein a is a positive integer in the range of 1 to 100 and b is a positive integer in the range of 1 to 20;
n is a positive integer, selected for PEG in the range of 4 to 300, for PPG in the range of 3 to 12 and for PEG-PPG block copolymer in the range of 3 to 140; or
R is a carbohydrate radical, selected from the monosaccharides glucose, fructose, ribose, desoxyribose, inositol, from the oligosaccharides saccharose, raffinose, gentianose, malecitose, stachyose, verbascose, from the polysaccharides starch, lichenins, glycogen, dextrins, dextrans, inulins, fructosans, lavans, mannans, galactans, xylans, arabans, pectins, macropolysaccharides, glycoproteins, from polyuridenylic acid, polyglucuronic acid, polygalacturonic acid, polymannuronic acid and/or alginic acid;
A is absent or
A is an alkyl, alkoxy, acyl, acylamine, alkylamine group, in which the number of carbon atoms in the alkoxy, acyl, acylamine, alkyl group lies in the range of 1 to 4;
B is a phosphorus-containing radical selected from monophosphate, diphosphate, polyphosphate, phosphonate, thiophosphate, thiophosphonate or a carboxylate-, sulphate-, sulphonate-, mercapto-, silanetriol or trialkoxysilane-containing radical;
(ii) the phosphate group-containing nucleotides mono-, di-, tri-phosphoric acid esters or mono-, di-, tri-phosphoric acid ester chlorides of adenosine, guanosine, cytidine, uridine, thymidine, desoxyadenosine, desoxyguanosine, desoxycytidine, desoxythymidine, inosine, pyrimidine, cytosine, uracil, thymine, purine, adenine, guanine, methylcytosine, 5-hydroxymethyl-cytosine, 2-methyladenine, 1-methylguanine, thiamine, flavin, riboflavin and pyridoxalphosphate, pyridoxamine phosphate, ribonucleic acid, ribonucleic acid sequences, desoxyribonucleic acid, desoxyribonucleic acid sequences;
(iii) the silicate group-containing compounds of orthosilic acid and the condensation products thereof; and/or
(iv) X - R - A - B is mercaptopurine, -cytosine, -guanine, -uracil, -thymine, -hypoxanthine and the mercapto-nucleosides thereof and the mercapto-desoxynucleosides thereof;
(v) X - R - A - B is a polyaminocarbohydrate.

5. Superparamagnetic particles according to one of Claims 1 to 4, characterized in that
(i) a tissue-specific binding substances from the group comprising antigens, antibodies, ribonucleic acids, desoxyribonucleic acids, ribonucleic acid sequences, desoxyribonucleic acid sequences, haptens, protein A, protein G, endotoxin-binding proteins, lectins, selectins;
(ii) a pharmacologically active substance from the group comprising antitumour proteins, enzymes, antitumour enzymes, antibiotics, vegetable alkaloids, alkylation reagents, antimetabolites, hormones and hormone antagonists, interleucines, interferons, growth factors, turmour necrosis factors, endotoxins, lymphotoxins, urokinases, streptokinases, plasminogen-streptokinase-activator-complex, tissueplasminogen-activators, desmodus-plasminogen-activators, macrophage-activator bodies, antisera, protease inhibitors and/or radioactive phosphorus ³²P-containing stabilizer substances, surfactants;
(iii) pharmacologically active cells from the group comprising organelles, viruses, microbes, algae, fungi, in particular erythrocytes, thrombocytes, granulocytes, monocytes, lymphocytes and/or Langerhans islets;
(iv) pharmacologically active complex forming agents from the group comprising polycarboxylic acids, aminocarboxylic acids, porphyrins, catecholamines;
(v) phosphate or phosphonate group-containing drugs; and/or
(vi) cell fusion facilitating substances
are chemically bound onto the superparamagnetic particles.

6. Process for producing superparamagnetic particles consisting of single-domain particles and stabilizer substances characterized in that superparamagnetic single-domain particles are produced from iron oxide, iron mixed oxide or iron with a particle size in the range between 3 and 20 nanometres by precipitation from aqueous iron salt solutions with lye or ammoniacal liquor in the pH range of 8.0 to 10.0, are adjusted to a pH of between 3 and 7 with an acid and are aggregated at a temperature in the range of 50 to 120°C and optionally elevated pressure in this pH and temperature range, to form aggregates having a particle size in the range between 10 and 1000 nanometres; the aggregates are purified in a known manner and are reacted with 20 to 50% by weight of stabilizer substances, the stabilizer substance being selected from the group comprising phosphate, diphosphate, carboxylate, polyphosphate, thiophosphate, phosphonate, thiophosphonate, sulphate, sulphonate, mercapto, silanetriol, trialkoxysilane group-containing polyalkylene glycols, the carbohydrates or the phosphate group-containing nucleotides, their oligomers or their polymers, which can have further binding sites; and the stable, decomposable aggregates with defined behaviour obtained are purified in a known manner in the magnetic field and are optionally further coupled in a known manner to pharmacologically or diagnostically active substances.

7. Pharmacologically active preparation, consisting of a pharmacologically acceptable carrier and superparamagnetic particles, consisting of superparamagnetic single-domain particles of iron oxide, iron mixed oxide or iron with a particle size in the range between 3 and 20 nanometres, which are combined to form stable, decomposable aggregates exhibiting defined behaviour in the magnetic field, with a particle size of the aggregates in the range between 10 and 1000 nanometres, wherein the aggregates have a monomolecular layer on their surface, consisting of stabilizer substances from the group comprising the phosphate, diphosphate, carboxylate, polyphosphate, thiophosphate, phosphonate, thiophosphonate, sulphate, sulphonate, mercapto, silanetriol, trialkoxysilane group-containing polyalkylene glycols, the carbohydrates or the phosphate group-containing nucleotides, their oligomers or their polymers, which can have further binding sites, optionally in conjunction with a tissue-specific binding substance, a pharmacologically active substance, a pharmacologically active cell, a pharmacologically active complex forming agent, a drug or a cell fusion-facilitating substance from the groups mentioned in claim 5.

8. Use of a pharmacologically active preparation according to Claim 7 for destroying tumours and increasing immunity, optionally under the influence of magnetic fields.

## Revendications

1. Particules supraparamagnétiques se composant de particules à un domaine et de substances stabilisantes caractérisées en ce que des particules supraparamagnétiques à un domaine prises parmi les oxydes de fer, les oxydes mixtes de fer ou le fer et présentant une granulométrie comprise entre 3 et 20 nanomètres sont associées en agrégats stables, dégradables et présentant un comportement défini dans le champ magnétique et dont la granulométrie est comprise entre 10 et 1000 nanomètres, ces agrégats présentant sur leur surface une couche monomoléculaire composée de substances stabilisantes prises dans le groupe des polyalkylène-glycols comprenant des groupements phosphates, diphosphates, carboxylates, polyphosphates, thiophosphates, phosphonates, thiophosphonates, sulfates, sulfonates, thioalcools, silanetriols, trialcoxysilanes, des hydrates de carbone ou des nucléotides comprenant des fonctions phosphates et dont les oligomères ou les polymères peuvent présenter d'autres points de fixation.

2. Particules supraparamagnétiques selon la revendication 1 caractérisées en ce que la granulométrie des particules à un domaine supraparamagnétiques est comprise entre 3 et 20 nm et la granulométrie des agrégats supraparamagnétiques entre 10 et 1000 nm.

3. Particules supraparamagnétiques selon la revendication 1 ou 2 caractérisées en ce que les particules à un domaine supraparamagnétiques se composent de γ-Fe₂O₃, d'oxydes mixtes de fer de formule générale MO·Fe₂O₃, M correspondant aux ions métalliques bivalents Fe, Mg, Be, Mn, Zn, Co, Ba, Sr, Cu ou à leurs mélanges, d'oxydes mixtes de fer de formule générale m Fe₂O₃ · nMe₂O₃, Me correspondant aux ions métalliques trivalents Al, Cr, à des métaux terreux rares ou à leurs mélanges, ou de fer.

4. Particules supraparamagnétiques selon l'une des revendications 1 à 3 caractérisées en ce que les substances stabilisantes sont choisies parmi
(i) les composés de formule générale
X - R - A - B
X représentant une fonction, choisie parmi les fonctions alcoxyle, monoalkylamine, dialkyleamine, trialkyleamine et alkylthio, pour lesquelles le nombre d'atomes de carbone dans la partie alcoyle de ces groupements est compris entre 1 et 4, ou représentant une fonction, choisie parmi les groupes hydroxyle, amine, aldéhyde, diméthylacétal, diéthylacétal époxy, thiol, carboxy, 4,6-dichlorotriazine, acide hydroxamique, isocyanate, acide d'acyle, anhydride, sel de diazonium, imonocarbonate, sulfonate de toluène;
R manque ou
R est un polyalkylèneglycol, un radical de glycol de prolypropylène miscible dans l'eau ou un radical de copolymérisat en bloc miscible dans l'eau composé de polyéthylène-glycol (PEG) et de polypropylène-glycol (PPG) choisis parmi les copolymérisats en bloc
(PEG)ₐ-(PEG)_{b}, (PEG)ₐ-(PPG)_{b}-(PEG)ₐ, (PPG)_{b}-(PEG)ₐ-(PPG)_{b}
a étant un nombre entier positif compris entre 1 et 100 et b un nombre entier positif compris entre et 20;
n est un nombre entier positif, choisi entre 4 et 300 pour le PEG, entre 3 et 12 pour le PPG et entre 3 et 140 pour le copolymérisat en bloc PEG-PPG; ou
R est un radical d'un hydrate de carbone, choisi parmi les monosaccharides glucose, fructose, ribose, désoxuribose, inositol, parmi les oligoholosides saccharose, raffinose, gentianose, malécitose, stachyose, verbascose, parmi les polysaccharides amidon, lichénine, glycogène, dextrine, dextrane, inuline, fructosane, lévane, mannane, galactane, xylane, arabane, pectine, macropolysaccharide, glycoprotéide, parmi les polyacides uridényles, les polyacides glucuroniques, les polyacides galacturoniques, les polyacides mannuroniques et/ou l'acide alginique;
A manque ou
A est un groupe alkyle, alkoxyle, acyle, acylamine, alkylamine pour lequel le nombre des atomes de carbone du groupe alkoxyle, acyle, acylamine, alcoyle est compris entre 1 et 4;
B est un radical phosphoré choisi parmi le monophosphate, diphosphate, polyphosphate, phosphonate, thiophosphate, thiophosphonate, ou un radical contenant du carboxylate du sulfate, du sulfonate, du thioalcool, du silanetriol ou du trialkoxylesilane;
(ii) les nucléotides contenant les groupes phosphates tels que le monoester phosphorique, le diester phosphorique, le triester phosphorique ou les chlorures de monoester, diester ou triester phosphorique d'adénosine, de guanosine, de cytidine, d'uridine, de thymidine, de désoxyadénosine, de désoxyguanosine, de désoxycytidine, de désoxythymidine, d'inosine, de pyrimidine, de cytosine, d'uracile, de thymine, de purine, d'adénine, de guanine, de méthylecytosine, de 5-hydroxyméthyle-cytosine, de 2-méthyleadénine, de 1-méthyleguanine, de thiamine, de flavine, de riboflavine, ainsi que le phosphate pyridoxal, le pyridoxaminophosphate, l'acide ribonucléique, les séquences d'acide ribonucléique, les acides désoxyribonucléiques, les séquences d'acide désoxyribonucléique;
(iii) les composés comprenant du silicate de l'acide silicique ortho et de ses condensats; et/ou
(iv) X - R - A - B est de la mercaptopurine, mercaptocytosine, mercaptoguanine, du mercaptouracile, de la mercaptothymine, de la mercaptohypoxanthine, ainsi que leur mercapto-nucléosides et leurs mercapto-désoxynucléosides;
(v) X - R - A - B est un hydrate de carbone polyamine.

5. Particules supraparamagnétiques selon l'une des revendications 1 à 4 caractérisées en ce qu'on combine l'une des substances suivantes sur les particules supraparamagnétiques:
(i) des substances de liaison spécifiques au tissu telles que des antigènes, des anticorps, des acides ribonucléiques, des acides désoxyribonucléiques, des fréquences acides ribonucléiques, des fréquences acides désoxyribonucléiques, des haptènes, des protéines γA, des protéines γG, des protéines de fixation des endotoxines, de la lectine, de la sélectine,
(ii) des substances à effet médicamenteux telles que les protéines anti-tumorales, les enzymes, les enzymes anti-tumorales, les antibiotiques, les alcaloïdes, les réactifs d'alkylation, les antagonistes métabolites, les hormones et les antagonistes des hormones, l'interleukine, l'interféron, les facteurs de croissance, les facteurs de nécrose de cellules cancéreuses, les endotoxines, les lymphotoxines, l'urokinase, la streptokinase, le complexe activateur plasminogène-streptokinase, les activateurs tissulaires-plasminogènes, les activateurs desmodus-plasminogène, les corps d'activation de macrophages, les antisérums, les inhibiteurs de protéases et/ou les substances stabilisantes contenant du phosphore ³²P radioactif, les agents tensioactifs,
(iii) des cellules pharmacologiquement actives telles que des organelles, des virus, des microbes, des algues, des champignons, notamment des érythrocytes, des thrombocytes, des granulocytes, des monocytes, des lymphocytes et/ou des îlots pancréatiques,
(iv) des agents complexants pharmacologiquement actifs du groupe des polyacides carboniques, aminoacides carboxyliques, porphyrines, catécholamines,
(v) des substances médicamenteuses comprenant des groupements phosphate ou phosphonate, et/ou
(vi) des agents de fusion cellulaire.

6. Procédé de fabrication de particules supraparamagnétiques composées de particules à un domaine et de substances stabilisantes, caractérisé en ce qu'on obtient des particules supraparamagnétiques d'oxyde de fer, d'oxyde mixte de fer ou de fer présentant une granulométrie comprise en 3 et 20 nanomètres par précipitation à partir de solutions aqueuses de sel ferreux avec une lessive alcaline ou de l'eau ammoniacale dans une gamme de pH allant de 8,0 à 10,0, le pH étant ramené entre 3 et 7 sous l'effet d'un acide, ces particules étant amenées, à une température comprise entre 50 et 120°C, éventuellement sous une pression augmentée et dans ces conditions de pH et de température, à s'agréger en agrégats dont la granulométrie est comprise entre 10 et 1000 nanomètres; ces agrégats étant nettoyés de façon connue en soi et pourvus de 20 à 50% en poids de substance stabilisante, la substance stabilisante étant choisie dans le groupe des polyalkylène-glycols comprenant des groupements phosphates, diphosphates, carboxylates, polyphosphates, thiophosphates, phosphonates, thiophosphonates, sulfates, sulfonates, thioalcools, silanetriols, trialcoxysilanes, des hydrates de carbone ou des nucléotides comprenant des fonctions phosphates et dont les oligomères ou les polymères peuvent présenter d'autres points de fixation; et les agrégats ainsi obtenus, lesquels sont stables, dégradables et à comportement défini dans un champ magnétique, étant nettoyés de façon connue en soi et éventuellement couplés de façon connue en soi avec des substances pharmacologiquement ou diagnostiquement actives.

7. Préparation pharmacologiquement active comprenant un support acceptable pharmacologiquement et des particules supraparamagnétiques se composant de particules à un domaine et de substances stabilisantes caractérisées en ce que des particules supraparamagnétiques à un domaine prises parmi les oxydes de fer, les oxydes mixtes de fer ou le fer et présentant une granulométrie comprise entre 3 et 20 nanomètres sont associées en agrégats stables, dégradables et présentant un comportement défini dans le champ magnétique et dont la granulométrie est comprise entre 10 et 1000 nanomètres, ces agrégats présentant sur leur surface une couche monomoléculaire composée de substances stabilisantes prises dans le groupe des polyalkylène-glycols comprenant des groupements phosphates, diphosphates, carboxylates, polyphosphates, thiophosphates, phosphonates, thiophosphonates, sulfates, sulfonates, thioalcools, silanetriols, trialcoxysilanes, des hydrates de carbone ou des nucléotides comprenant des fonctions phosphates et dont les oligomères ou les polymères peuvent présenter d'autres points de fixation, éventuellement en composition avec une substance de liaison spécifique au tissu, une substance pharmacologiquement active, une cellule pharmacologiquement active, un agent complexant pharmacologiquement actif, un substance médicamenteuse ou un agent de fusion cellulaire pris dans les groupes indiqués dans la revendication 5.

8. Utilisation d'une préparation pharmacologiquement active selon la revendication 7 à des fins de détérioration de cellules tumorales et d'immuno-stimulation, sous l'effet ou non de champs magnétiques.
